(19) Europäisches Patentamt / European Patent Office / Office européen des brevets

(11) EP 4 484 427 A1

(12) **EUROPEAN PATENT APPLICATION**
published in accordance with Art. 153(4) EPC

(43) Date of publication:
**01.01.2025 Bulletin 2025/01**

(21) Application number: **23759274.6**

(22) Date of filing: **24.02.2023**

(51) International Patent Classification (IPC):
*C07D 471/04* (2006.01) *A61K 31/437* (2006.01)
*A61P 25/16* (2006.01) *A61P 25/28* (2006.01)
*A61P 35/00* (2006.01)

(52) Cooperative Patent Classification (CPC):
**A61K 31/437; A61P 25/16; A61P 25/28; A61P 35/00; C07D 471/04**

(86) International application number:
**PCT/CN2023/078130**

(87) International publication number:
**WO 2023/160652 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.02.2022 CN 202210178818**
**28.04.2022 CN 202210470959**

(71) Applicant: **Suzhou Yabao Pharmaceutical R&D Co., Ltd.**
**Suzhou, Jiangsu 215123 (CN)**

(72) Inventors:
- **SHAO, Qing**
  **Suzhou, Jiangsu 215123 (CN)**
- **HUANG, Zhijiang**
  **Suzhou, Jiangsu 215123 (CN)**
- **ZHUO, Lang**
  **Suzhou, Jiangsu 215123 (CN)**

(74) Representative: **Gille Hrabal Partnerschaftsgesellschaft mbB**
**Patentanwälte**
**Brucknerstraße 20**
**40593 Düsseldorf (DE)**

# (54) SUBSTITUTED FUSED RING COMPOUND, PHARMACEUTICAL COMPOSITION THEREOF, PREPARATION METHOD THEREFOR AND USES THEREOF

(57) Provided are a substituted fused ring compound represented by formula (I), a pharmaceutical composition thereof, a preparation method therefor and the uses thereof. The compound has a good LRRK2 kinase regulation/inhibition effect, and thus can be used for treating symptoms and diseases related to LRRK2 kinase activity, and can be used for preparing drugs for such symptoms and diseases.

EP 4 484 427 A1

## Description

The present application claims priority to Chinese Patent Application No. 202210178818.7 filed to China National Intellectual Property Administration on Feb. 25, 2022 and entitled "SUBSTITUTED FUSED RING COMPOUND, PHARMACEUTICAL COMPOSITION THEREOF, PREPARATION METHOD THEREFOR AND USES THEREOF", and Chinese Patent Application No. 202210470959.6 filed to China National Intellectual Property Administration on Apr. 28, 2022 and entitled "SUBSTITUTED FUSED RING COMPOUND, PHARMACEUTICAL COMPOSITION THEREOF, PREPARATION METHOD THEREFOR AND USES THEREOF", the contents of which are incorporated herein by reference in their entireties.

## TECHNICAL FIELD

The present disclosure belongs to the field of LRRK2 kinase drugs, and particularly relates to a substituted fused ring compound, a pharmaceutical composition thereof, a preparation method therefor, and use thereof.

## BACKGROUND

Parkinson's disease (PD) is a common neurodegenerative disease that affects 1-2% of the elderly. Genome-wide association studies (GWAS) have identified, in non-familial PD, 24 loci associated with 28 related genetic risk variations. Among them, mutations in LRRK2 (Park8) are also found in genetic forms, identifying common molecular pathway-driven pathogenesis of both familial and non-familial PD, the most common cause of related diseases (Simon-Sanchez J, Schulte C, Bras JM, Sharma M, Gibbs JR, et al., Genome-wide association study reveals genetic risk underlying Parkinson's disease. Nat Genet, 2009, 41, 1308-1312). The profile of PD pathogenic mutations in LRRK2 is mainly associated with the kinase (G2019S, I2020T) and the ROC-COR domain (R1441C/G/H, Y1699C), which means that these enzymatic activities are critical for disease development. Pathogenic mutations are rare, found in about 2% of the overall population. However, the most common mutation G2019S is found in up to 40% of patients in some ethnic groups, activating the kinase to increase its activity by two to three times (West AB, Moore DJ, Biskup S, Bugayenko A, Smith WW, et al., From The Cover: Parkinson's disease-associated mutations in leucine-rich repeat kinase 2 augment kinase activity. Proceedings of the National Academy of Sciences, 2005, 102, 16842-16847). In addition to pathogenic mutations, a common genetic variation in LRRK2 is a risk factor for sporadic PD (Tan, E. K. Identification of a common genetic risk variant (LRRK2 Gly2385Arg) in Parkinson's disease. Ann Acad Med Singapore. 2006, 35, 840-842).

In 2004, LRRK2 was identified as the gene responsible for PD inheritance, which was associated with the PARK8 locus and it was found to consist of 51 exons, resulting in a protein having a high molecular weight (268kDa). Subsequently, a number of primary structural variants of LRRK2 were identified, including dominant mutations separated from familial PD that also occur in sporadic PD, as well as polymorphisms at the LRRK2 loci that increase the lifetime risk of sporadic PD development.

LRRK2 is a multi-domain protein with two enzymatic functions at its core. The GTPase domain comprises the Ras of complex protein (ROC) which terminates in a spacer domain at the C-terminal of Roc domain (COR), followed by a kinase domain belonging to serine/threonine kinases. The enzymatic core is surrounded by protein-protein interaction domains, including the N-terminal leucine-rich repeat (LRR) domain of Armadillo, ankyrin, and LRRK2. The C-terminal of LRRK2 contains WD40 domain which is a region essential for protein folding and can control the functions and kinase activities of LRRK2 (Rudenko IN, Kaganovich A, Hauser DN, Beylina A, Chia R, et al., The G2385R variant of leucine-rich repeat kinase 2 associated with Parkinson's disease is a partial loss-of-function mutation. Biochemical Journal, 2012, 446, 99-111). Interestingly, the newly-described dominant pathogenic mutation is found in the enzymatic nucleus of LRRK2, indicating that the modification of LRRK2 activity greatly affects the development and progression of PD.

To date, nearly 40 single amino acid substitution mutations in the LRRK2 mutations have been associated with autosomal dominant PD. In European, the most common mutant form of LRRK2 comprises about 6% of familial PD cases and 3% of sporadic PD cases, including the amino acid substitution of Gly2019 with a Ser residue. Gly2019 is located within the conserved DYG-$Mg^{2+}$ binding motif in subdomain-VII of the kinase domain. Recent reports have shown that this mutation enhances the autophosphorylation of LRRK2 and increases its ability to phosphorylate myelin basic protein 2-3 fold. Both of cytotoxicity and inclusion body formation are observed when G2019S-LRRK2 is overexpressed in cell lines and primary neuronal cultures, regardless of the presence of oxidative stress. These results, together with the genetic inactivation of LRRK2 kinase activity, show a protective effect on the toxic phenotype, which suggests that changes in LRRK2 kinase activity may be associated with neurotoxicity and pathogenic mechanisms of LRRK2-PD.

Studies have found that induced pluripotent stem cells (iPSCs) from LRRK2 G2019S patients with Parkinson's disease exhibit deficient neurite outgrowth and increased sensitivity to rotenone, which can be improved by genetic modification of the G2019S mutation or treatment of the cells with small molecule inhibitors of LRRK2 kinase activity (Reinhardt P, Schmid B, Burbulla Lena F, Schöndorf David C, Wagner L, et al., Genetic Correction of a LRRK2 Mutation in

Human iPSCs Links Parkinsonian Neurodegeneration to ERK-Dependent Changes in Gene Expression. Cell Stem Cell, 2013, 12, 354-367). The increased mitochondrial damage associated with LRRK2 G2019S mutations in iSPC is also blocked by genetic correction of the G2019S mutation.

## SUMMARY

In order to ameliorate the technical problems described above, the present disclosure provides a compound represented by formula (I), or a racemate, a stereoisomer, a tautomer, an isotopically labeled compound, an N-oxide, a hydrate, a solvate, a polymorph, a metabolite, a pharmaceutically acceptable salt, a pharmaceutically acceptable ester, or a prodrug compound thereof

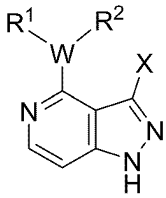

(I)

wherein,

W is selected from N or O;

$R^1$ is selected from the following groups unsubstituted or substituted with one, two, or more $R^a$: $C_{1-10}$ alkyl, $C_{3-10}$ cycloalkyl, and 3- to 10-membered heterocyclyl;

each $R^a$ is identical or different and is independently selected from halogen, hydroxyl, $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

or alternatively, two $R^a$, together with the atom(s) to which they are attached, form the following groups unsubstituted or optionally substituted with one, two, or more $R^c$: $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

$R^2$ is absent or selected from H or $C_{1-10}$ alkyl, wherein, when W is O, $R^2$ is absent; when W is N, $R^2$ is selected from H or $C_{1-10}$ alkyl;

or alternatively, $R^1$ and $R^2$, together with W, form 3- to 10-membered heterocyclyl unsubstituted or optionally substituted with one, two, or more $R^b$;

each $R^b$ is identical or different and is independently selected from halogen, hydroxyl, $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

or alternatively, two $R^b$, together with the atom(s) to which they are attached, form the following groups unsubstituted or optionally substituted with one, two, or more $R^c$: $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

each $R^c$ is identical or different and is independently selected from halogen, hydroxyl, $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

X is selected from 5- to 10-membered heteroaryl substituted with one, two, or more $R^3$;

each $R^3$ is identical or different and is independently selected from the following groups unsubstituted or substituted with one, two, or more $R^4$: $C_{1-10}$ alkyl, $C_{3-10}$ cycloalkyl, 3- to 10-membered heterocyclyl, and 5- to 10-membered heteroaryl;

or alternatively, two $R^3$, together with the atom(s) to which they are attached, form the following groups unsubstituted or optionally substituted with one, two, or more $R^4$: $C_{3-10}$ cycloalkyl and 3- to 10-membered heterocyclyl;

each $R^4$ is identical or different and is independently selected from halogen, hydroxyl, cyano, or the following groups unsubstituted or substituted with one, two, or more $R^d$: $C_{1-10}$ alkyl, $C_{1-10}$ alkylene, $C_{3-10}$ cycloalkyl, 3- to 10-membered heterocyclyl, and 5- to 10-membered heteroaryl, wherein $C_{1-6}$ alkylene means that the carbon atom in $R^4$, together with the carbon atom to which $R^3$ is attached, forms a double bond;

each $R^d$ is identical or different and is independently selected from halogen, hydroxyl, cyano, $C_{1-6}$ alkyl, $C_{1-6}$ alkyloxy, $C_{3-10}$ cycloalkyl, $C_{3-10}$ cycloalkyloxy, 3- to 10-membered heterocyclyl, 3- to 10-membered heterocyclyloxy, 5- to 10-membered heteroaryl, and 5- to 10-membered heteroaryl oxy.

According to an embodiment of the present disclosure, $R^1$ is selected from the following groups unsubstituted or substituted with one, two, or more $R^a$: $C_{1-8}$ alkyl, $C_{3-8}$ cycloalkyl, and 3- to 8-membered heterocyclyl;
for example, $R^1$ is selected from the following groups unsubstituted or substituted with one, two, or more $R^a$: $C_{1-6}$ alkyl, $C_{3-7}$ cycloalkyl, and 3- to 7-membered heterocyclyl;

According to an embodiment of the present disclosure, each $R^a$ is identical or different and is independently selected from F, Cl, Br, and $C_{1-6}$ alkyl;
as an example, $R^1$ is selected from

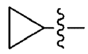 , 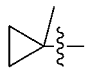 , 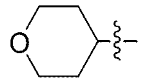 , 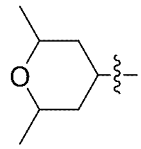 ,

$-CH_2CF_3$, and $-CH(CH_3)CF_3$.

According to an embodiment of the present disclosure, $R^1$ and $R^2$, together with W, form the following groups unsubstituted or optionally substituted with one, two, or more $R^b$:

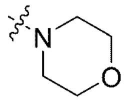 ,

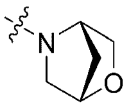 , 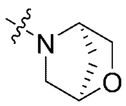 , 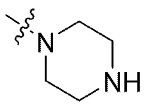 , and 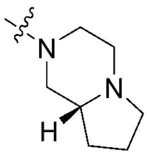 .

According to an embodiment of the present disclosure, X is selected from 5- to 6-membered heteroaryl substituted with one, two, or more $R^3$;
for example, X is selected from

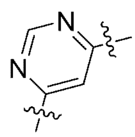 and 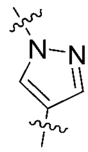

substituted with one, two, or more $R^3$;
as an example, X is selected from

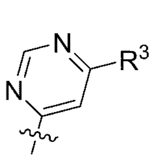 and 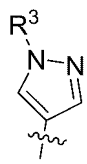 .

According to an embodiment of the present disclosure, $R^3$ is selected from the following groups unsubstituted or substituted with one, two, or more $R^4$: $C_{1-6}$ alkyl, $C_{3-8}$ cycloalkyl, 3- to 8-membered heterocyclyl, and 5- to 6-membered heteroaryl;

for example, $R^3$ is selected from the following groups unsubstituted or substituted with one, two, or more $R^4$: methyl, ethyl, propyl, isopropyl, cyclopropyl, cyclobutyl,

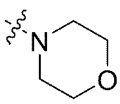 , 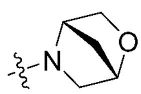 ,

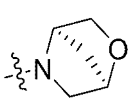, 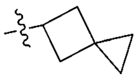, 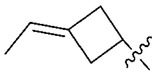, 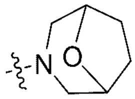, 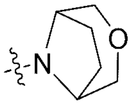, and 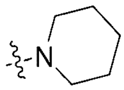;

each $R^4$ is identical or different and is independently selected from fluorine, chlorine, bromine, hydroxyl, cyano, or the following groups unsubstituted or substituted with one, two, or more $R^d$: $C_{1-6}$ alkyl, $C_{1-6}$ alkylene, $C_{3-8}$ cycloalkyl, and 3- to 8-membered heterocyclyl;

as an example, $R^3$ is selected from isopropyl,

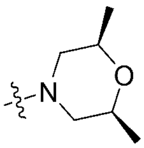, 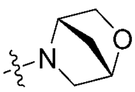, 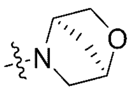, 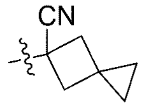,

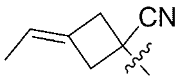, 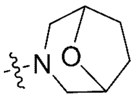, 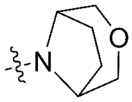, 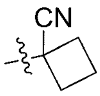, 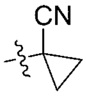, 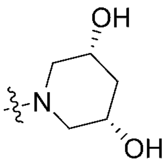, and 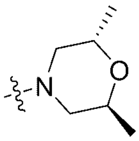.

According to a preferred embodiment of the present disclosure, the compound represented by formula (I) is preferably a compound represented by the following formula (II):

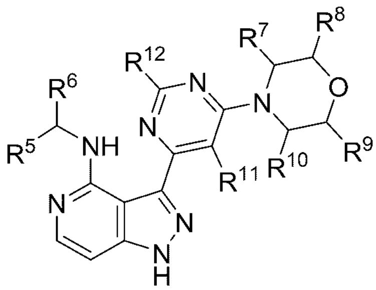

(II)

wherein $R^5$ is selected from $C_{1-6}$ alkyl substituted with one, two, or more halogens, preferably $C_{1-6}$ alkyl substituted with 1, 2, 3, 4, 5, 6, 7, 8, 9, or 10 halogens; for example, methyl or ethyl substituted with 1, 2, 3, 4, or 5 fluorine or chlorine atoms, e.g., trifluoromethyl;

$R^6$ is selected from $C_{1-6}$ alkyl, e.g., methyl, ethyl, propyl, or isopropyl;

$R^7$, $R^8$, $R^9$, and $R^{10}$ are identical or different and are each independently selected from H or $C_{1-6}$ alkyl, e.g., H, methyl, ethyl, propyl, or isopropyl;

$R^{11}$ and $R^{12}$ are identical or different and are each independently selected from H or $C_{1-6}$ alkyl, e.g., H, methyl, ethyl, propyl, or isopropyl.

According to a preferred embodiment of the present disclosure, the compound represented by formula (I) is preferably a compound represented by the following formula (III):

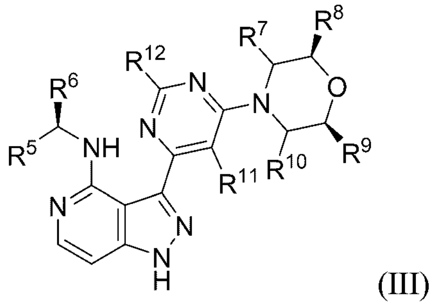

(III)

wherein $R^5$, $R^6$, $R^7$, $R^8$, $R^9$, $R^{10}$, $R^{11}$, and $R^{12}$ are defined as described above.

According to an exemplary embodiment of the present disclosure, the compound represented by formula (I) may be selected from the following compounds:

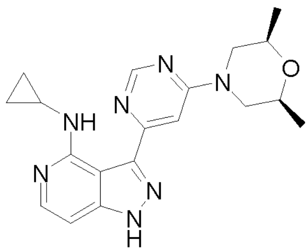

390

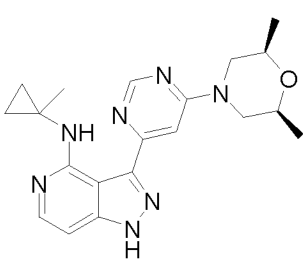

391

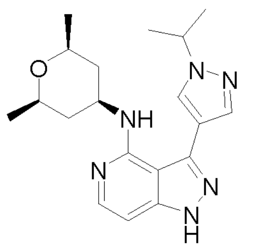

395

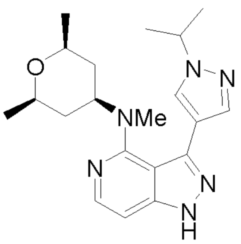

396

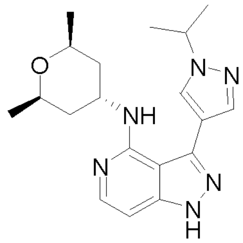

404

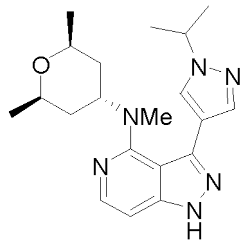

405

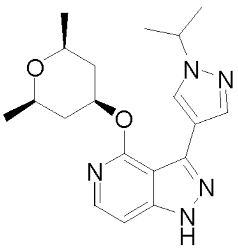

397

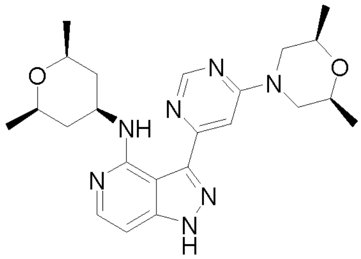

407

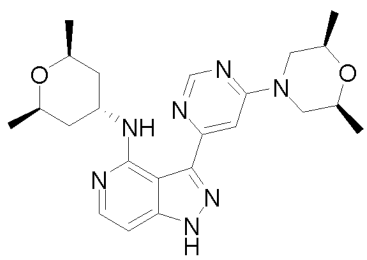

421

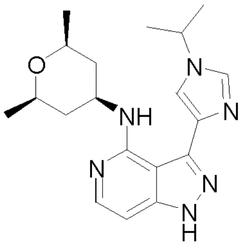

422

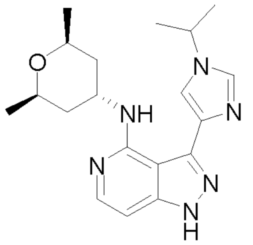

445

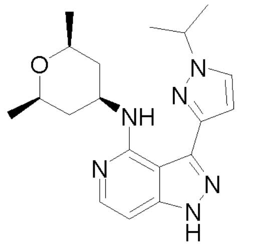

423

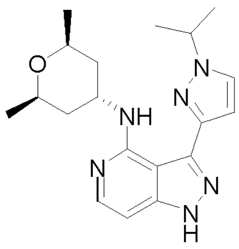

439

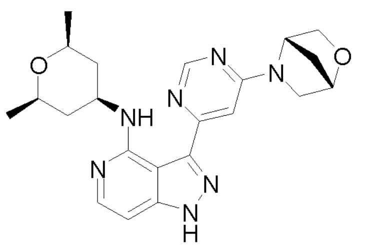

424

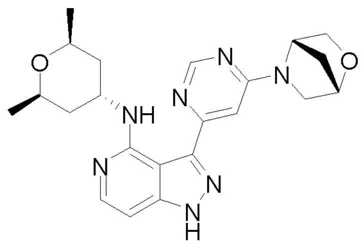

438

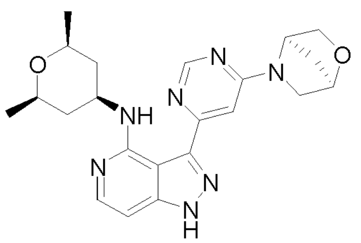

425

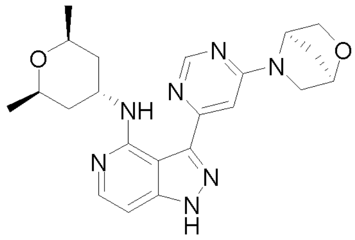

440

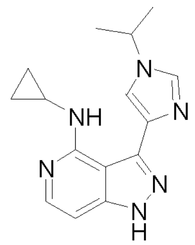

426

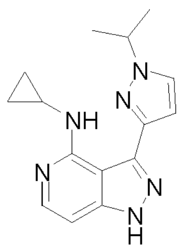

426

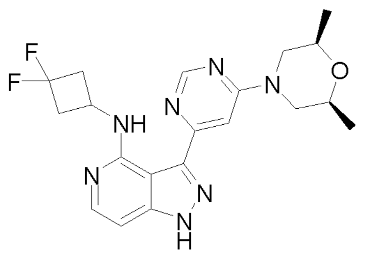

446

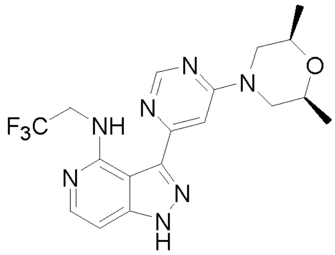

447

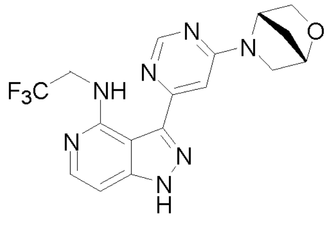

486

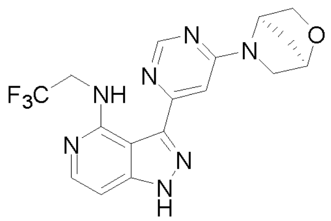

487

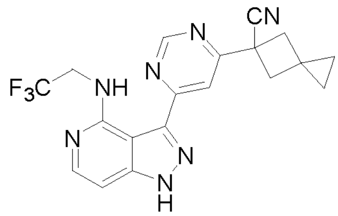

508

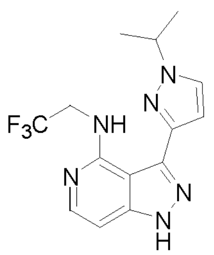

515

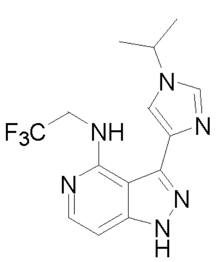

516

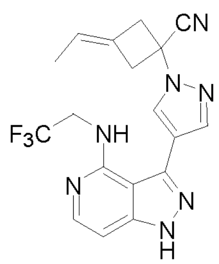

521

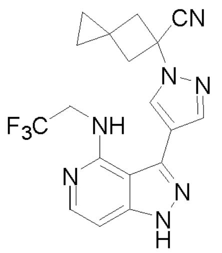

509

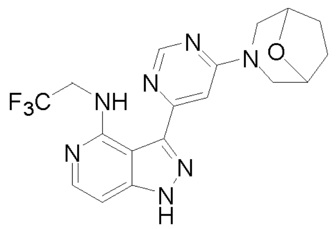

519

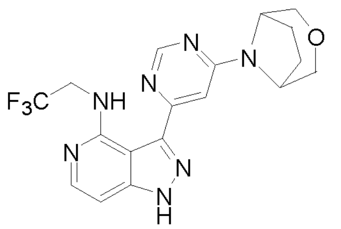

520

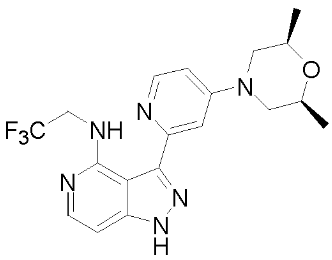

522

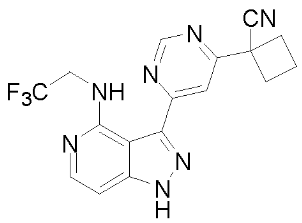

523

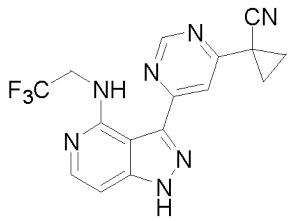

524

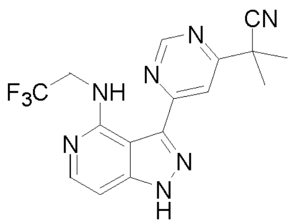

525

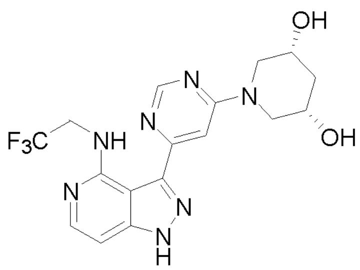

526

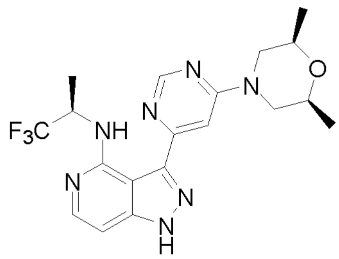

527

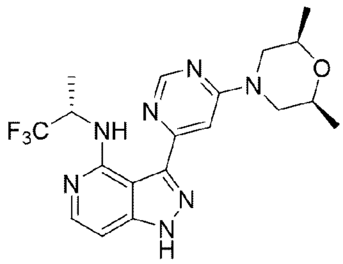

528

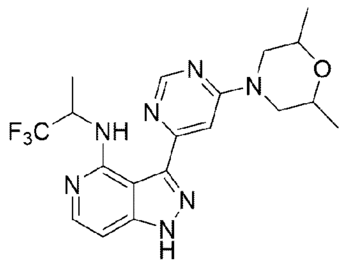

5278

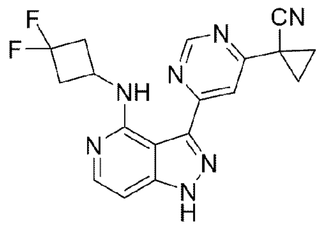

529

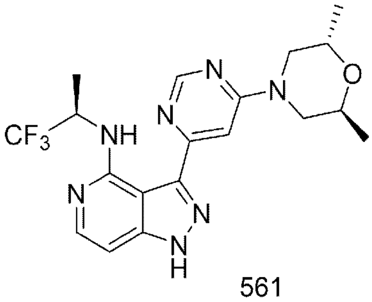

561

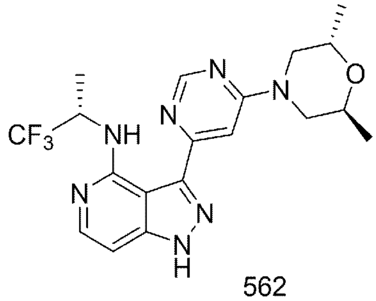

562

The present disclosure further provides a preparation method for the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug

compound thereof, wherein the method comprises the following reaction:

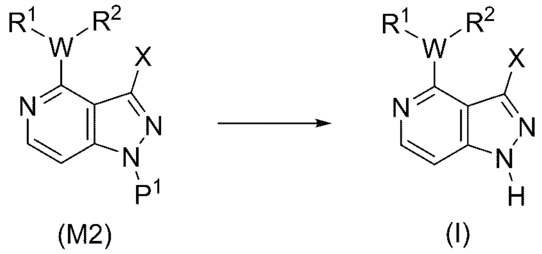

wherein $R^1$, $R^2$, W, and X are defined as described above; and $P^1$ is an amino protective group.

Preferably, the compound represented by formula (M2) is reacted under the condition of removing the protective group $P^1$ to obtain the compound represented by formula (I).

According to an embodiment of the preparation method described herein, $P^1$ is an amino protective group known to those skilled in the art. For example, a suitable amino protective group may be selected from, such as, alkyloxycarbonyl (e.g., *tert*-butoxycarbonyl, etc.), cycloalkylalkyloxycarbonyl (e.g., cyclohexylmethoxycarbonyl), substituted alkyloxycarbonyl (e.g., trichloroethoxycarbonyl, etc.), substituted arylalkyloxycarbonyl (e.g., *p*-nitrobenzyloxycarbonyl), arylalkyl (e.g., trityl or benzhydryl), heteroarylalkyl (e.g., pyridinylalkyl, such as 2-pyridinylmethyl and 4-pyridinylmethyl), and alkoxyarylalkyl (e.g., alkoxyphenylalkyl, such as *p*-methoxybenzyl, which may be abbreviated as "PMB").

According to an embodiment of the preparation method described herein, a preparation method for the compound represented by formula (M2) is further provided, comprising the following reaction:

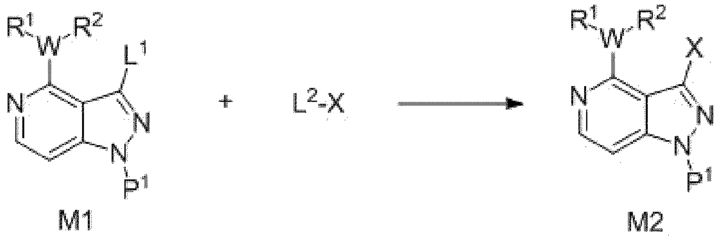

wherein $R^1$, $R^2$, W, X, and $P^1$ are defined as described above;

$L^1$ and $L^2$ are identical or different and are each independently selected from a leaving group capable of undergoing a coupling reaction.

According to an embodiment of the present disclosure, the preparation method for the compound represented by formula (M2) may be performed through a coupling reaction known in the art, for example, through a Stille coupling reaction or a Suzuki coupling reaction.

As a Stille coupling reaction or a Suzuki coupling reaction known to those skilled in the art, $L^1$ and $L^2$ that meet the needs of the reaction thereof are known to those skilled in the art. For example, $L^1$ may be selected from halogen, e.g., iodine; $L^2$ may be selected from alkyl tin groups or borate groups.

The present disclosure further provides a pharmaceutical composition comprising at least one of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof.

The present disclosure further provides a pharmaceutical composition comprising a therapeutically effective amount of at least one of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof.

According to an embodiment of the present disclosure, the pharmaceutical composition further comprises one or more pharmaceutically acceptable auxiliary materials.

According to an embodiment of the present disclosure, the pharmaceutical composition may further comprise one or more additional therapeutic agents.

According to an embodiment of the present disclosure, the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof, or the pharmaceutical composition may be used to modulate the activity of LRRK kinase,

particularly LRRK2 kinase. According to an embodiment of the present disclosure, the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof, or the pharmaceutical composition may be used to prevent and/or treat a disease or disorder, wherein the disease or disorder may include one, two, or more selected from neurodegenerative diseases, proliferative diseases, diseases associated with protein kinases, lysosomal diseases, Tau disease, and diseases caused by decreased dopamine levels, such as cancer, Parkinson's disease (PD) associated with GBA mutations, other α-synucleinopathies, tau protein disease, Alzheimer's disease, Gaucher disease, Niemann-Pick type C (NPC), argyrophilic grain disease, Pick's disease, corticobasal degeneration, progressive supranuclear palsy, frontotemporal dementia with parkinsonism linked to chromosome 17 (FTDP-17), withdrawal symptoms/relapse associated with drug addiction, and the like.

The present disclosure further provides a method for modulating the activity of LRRK kinase, particularly LRRK2 kinase, wherein the method comprises administering to a patient a prophylactically or therapeutically effective amount of at least one of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof.

According to an embodiment of the present disclosure, the LRRK kinase is preferably LRRK2 kinase, or a mutant or an isoform thereof, or a combination thereof.

The present disclosure further provides at least one of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof, or the pharmaceutical composition for use in diseases or disorders associated with the activity of LRRK kinase, particularly LRRK2 kinase.

According to an embodiment of the present disclosure, the LRRK kinase is preferably LRRK2 kinase, or a mutant or an isoform thereof, or a combination thereof.

The diseases or disorders associated with the activity of LRRK kinase, particularly LRRK2 kinase, include one, two or more selected from neurodegenerative diseases, proliferative diseases, diseases associated with protein kinases, lysosomal diseases, Tau disease, and diseases caused by decreased dopamine levels, such as cancer, Parkinson's disease (PD) associated with GBA mutations, other α-synucleinopathies, tau protein disease, Alzheimer's disease, Gaucher disease, Niemann-Pick type C (NPC), argyrophilic grain disease, Pick's disease, corticobasal degeneration, progressive supranuclear palsy, frontotemporal dementia with parkinsonism linked to chromosome 17 (FTDP-17), withdrawal symptoms/relapse associated with drug addiction, and the like.

The present disclosure further provides a method for preventing and/or treating a disease or disorder, wherein the method comprises administering to a patient at least one of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof; wherein the disease or disorder can include one, two, or more selected from neurodegenerative diseases, proliferative diseases, diseases associated with protein kinases, lysosomal diseases, Tau disease, and diseases caused by decreased dopamine levels, such as cancer, Parkinson's disease (PD) associated with GBA mutations, other α-synucleinopathies, tau protein disease, Alzheimer's disease, Gaucher disease, Niemann-Pick type C (NPC), argyrophilic grain disease, Pick's disease, corticobasal degeneration, progressive supranuclear palsy, frontotemporal dementia with parkinsonism linked to chromosome 17 (FTDP-17), withdrawal symptoms/relapse associated with drug addiction, and the like.

In some embodiments, the patient of the disease or disorder is a human.

The present disclosure further provides a method for treating or preventing a disease or disorder associated with the activity of LRRK kinase, particularly LRRK2 kinase, wherein the method comprises administering to a patient at least one of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof.

The present disclosure further provides use of at least one of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof for manufacturing a medicament.

The medicament may be used to prevent and/or treat a disease or disorder associated with the activity of LRRK kinase, particularly LRRK2 kinase.

Alternatively, the medicament may be used to prevent and/or treat a disease or disorder, wherein the disease or disorder can comprise one, two, or more selected from neurodegenerative diseases, proliferative diseases, diseases associated with protein kinases, lysosomal diseases, Tau disease, and diseases caused by decreased dopamine levels,

such as cancer, Parkinson's disease (PD) associated with GBA mutations, other α-synucleinopathies, tau protein disease, Alzheimer's disease, Gaucher disease, Niemann-Pick type C (NPC), argyrophilic grain disease, Pick's disease, corticobasal degeneration, progressive supranuclear palsy, frontotemporal dementia with parkinsonism linked to chromosome 17 (FTDP-17), withdrawal symptoms/relapse associated with drug addiction, and the like.

When used as a medicament, the compound of the present disclosure can be administered in the form of a pharmaceutical composition. Those compositions can be manufactured in a manner well known in the pharmaceutical arts and can be administered by a variety of routes depending on whether local or systemic treatment is desired and the area to be treated. The pharmaceutical composition can be administered topically (e.g., by transdermal, dermal, ocular and mucosal routes, including intranasal, vaginal and rectal administration routes), pulmonarily (e.g., by inhalation or insufflation of powders or aerosols, including by nebulizer; by intratracheal and intranasal routes), orally or parenterally. The parenteral administration includes intravenous, intra-arterial, subcutaneous, intraperitoneal or intramuscular injection or infusion; or intracranial (e.g., intrathecal or intracerebroventricular) administration. The pharmaceutical composition can be administered parenterally in a single large dose form, or can be administered by, for example, continuous infusion pump. The pharmaceutical composition and formulation administered topically can include a transdermal patch, an ointment, a lotion, a cream, a gel, a drop, a suppository, a spray, a liquid, a powder formulation, and a powder. Conventional pharmaceutical carriers, water, powders or oily bases, thickeners, and the like may be necessary or desirable.

In manufacturing the composition of the present disclosure, the active ingredient is typically mixed with an excipient, diluted with an excipient or enclosed within such a carrier as capsules, sachets, paper, or other container forms. When used as a diluent, the excipient may be a solid, semi-solid or liquid substance that serves as a vehicle, a carrier, or a medium for the active ingredient. Thus, the composition may be in the following forms: tablets, pills, powders, lozenges, sachets, cachets, elixirs, suspensions, emulsions, solutions, syrups, aerosols (solid or dissolved in a liquid vehicle); ointments containing, for example, up to 10% by weight of the active compound, soft and hard gelatin capsules, suppositories, sterile solutions for injection, and sterile packaged powders.

Certain examples of suitable excipients include lactose, glucose, sucrose, sorbitol, mannitol, starch, acacia, calcium phosphate, alginate, tragacanth, gelatin, calcium silicate, microcrystalline cellulose, polyvinylpyrrolidone, cellulose, water, syrup, and methylcellulose. The formulation may further contain lubricants such as talc, sodium stearate, magnesium stearate, sodium oleate, sodium benzoate, sodium acetate, sodium chloride and mineral oil; wetting agents; emulsifiers and suspending agents; preservatives such as methyl benzoate and hydroxypropyl benzoate; and sweetening agents and flavoring agents. The composition of the present disclosure can be formulated by the methods known in the art so as to provide immediate, sustained, or delayed release of the active ingredient after administration to the patient.

The pharmaceutical composition can be formulated in unit dosage forms, and each unit dosage form may contain about 1-1000 mg, for example, about 5-1000 mg, more typically about 5-500 mg, e.g., 5-100 mg, 100-500 mg, or 50-200 mg of the active ingredient. As an example, each dose may contain 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 200 mg, 210 mg, 220 mg, 230 mg, 240 mg, 250 mg, 260 mg, 270 mg, 280 mg, 290 mg, or 300 mg of the active ingredient. The term "unit dosage form" refers to physically discrete single dosage units suitable for use in human patients and other mammals, each unit containing a predetermined amount of active substance mixed with a suitable pharmaceutical excipient that can produce the desired therapeutic effect by calculation.

The active compound may be effective in a wide range of doses and is generally administered in a pharmaceutically effective amount. However, it will be understood that the amount of the compound actually administered is usually determined by a physician, in the light of the relevant circumstances, including the disorder to be treated, the selected route of administration, the compound actually administered; the age, weight, and response of an individual patient; the severity of the patient's symptoms and the like.

The therapeutic dosage of the compound of the present disclosure can be determined, for example, according to: the specific use of the treatment, the route of administration of the compound, the health and condition of the patient, and the judgment of the prescriber. The proportion or concentration of the compound of the present disclosure in the pharmaceutical composition may vary depending on a variety of factors including the dosage, chemical properties (e.g., hydrophobicity), and the route of administration. For example, at least one of the compound of the present disclosure, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof may be provided through a physiological aqueous buffer solution containing about 0.1-10% (w/v) of the compound for use in parenteral administration. Certain typical doses range from about 1 μg/kg to about 1 g/kg body weight/day. In certain embodiments, the doses range from about 0.01 mg/kg to about 100 mg/kg body weight/day, e.g., 0.1 mg/kg body weight/day, 0.2 mg/kg body weight/day, 0.3 mg/kg body weight/day, 0.4 mg/kg body weight/day, 0.5 mg/kg body weight/day, 0.6 mg/kg body weight/day, 0.7 mg/kg body weight/day, 0.8 mg/kg body weight/day, 0.9 mg/kg body weight/day, 1 mg/kg body weight/day, 5 mg/kg body weight/day, 10 mg/kg body weight/day, 15 mg/kg body

weight/day, 20 mg/kg body weight/day, 25 mg/kg body weight/day, 30 mg/kg body weight/day, 35 mg/kg body weight/day, 40 mg/kg body weight/day, 45 mg/kg body weight/day, or 50 mg/kg body weight/day. As an example, when the patient is a human, the dose administered to the patient may be 57 mg/70 kg to 171 mg/70 kg. The dosage is likely dependent on such variables as the type and degree of progression of the disease or disorder, the general health condition of a particular patient, the relative biological potency of the compound selected, the excipient formulation, and its route of administration. Effective dosage can be extrapolated from dose-response curves derived from *in vitro* or animal model test systems.

In manufacturing solid compositions such as tablets, the main active ingredient is mixed with pharmaceutical excipients to form a solid preformulation composition of a homogeneous mixture comprising the compound of the present disclosure. When referring to these preformulation compositions as homogeneous, it means that the active ingredient is generally distributed evenly throughout the composition such that the composition can be readily divided into equally effective unit dosage forms such as tablets, pills and capsules. The solid preformulation is then divided into unit dosage forms of the type described above containing, for example, about 0.1-1000 mg of the active ingredient of the present disclosure, and each unit dosage form can contain about 1-1000 mg, for example, about 5-1000 mg, more typically about 5-500 mg, e.g., 5-100 mg, 100-500 mg, or 50-200 mg of active ingredient. As an example, each dose can contain 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 200 mg, 210 mg, 220 mg, 230 mg, 240 mg, 250 mg, 260 mg, 270 mg, 280 mg, 290 mg, or 300 mg of active ingredient.

The tablets or pills of the present disclosure can be coated or compounded to obtain a dosage form affording the advantage of long-acting effect. For example, the tablets or pills contain an inner dosage component and an outer dosage component, the latter being in the form of an envelope of the former. The two components can be separated by an enteric-coated layer which serves to prevent the disintegration in the stomach to allow the inner component to pass through the duodenum entirely or to be delayed in release. A variety of substances can be used for such enteric-coated layers or coatings, including various polymeric acids and mixtures of polymeric acids and such substances as shellac, cetyl alcohol and cellulose acetate.

Liquid forms for oral administration or injection administration in which the compound and composition of the present disclosure can be incorporated include aqueous solutions, suitable flavoring syrups, aqueous or oil suspensions; and emulsions flavored with edible oils such as cottonseed oil, sesame oil, coconut oil or peanut oil; as well as elixirs and similar pharmaceutical vehicles.

Compositions for inhalation or insufflation include solutions and suspensions in pharmaceutically acceptable aqueous or organic solvents, or mixtures thereof, and powders. The liquid or solid compositions can contain suitable pharmaceutically acceptable excipients as described above. In certain embodiments, the composition is administered by the oral or nasal inhalation route for local or systemic effect. The composition can be nebulized using an inert gas. The nebulized solution can be inhaled directly from the nebulizing device or the nebulizing device can be attached to a face mask or an intermittent positive pressure ventilator. The solution, suspension, or powder compositions can be administered orally, or nasally by means of a device which can deliver the formulation in a suitable manner.

The amount of the compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof, or the pharmaceutical composition administered to a patient is varied, depending on the drug administered, the purpose of the administration, e.g., prevention or treatment, the condition of the patient, the mode of administration, etc. In therapeutic applications, the composition can be administered to a patient suffering from a disease in an amount sufficient to cure or at least partially inhibit the symptoms of the disease and its complications. The effective dosage may depend on the disease state being treated and the determination of the attending clinician depending on factors such as the severity of the disease, the age, weight and general condition of the patient, and the like. Certain typical doses range from about 1 μg/kg to about 1 g/kg body weight/day. In certain embodiments, the doses range from about 0.01 mg/kg to about 100 mg/kg body weight/day, e.g., 0.1 mg/kg body weight/day, 0.2 mg/kg body weight/day, 0.3 mg/kg body weight/day, 0.4 mg/kg body weight/day, 0.5 mg/kg body weight/day, 0.6 mg/kg body weight/day, 0.7 mg/kg body weight/day, 0.8 mg/kg body weight/day, 0.9 mg/kg body weight/day, 1 mg/kg body weight/day, 5 mg/kg body weight/day, 10 mg/kg body weight/day, 15 mg/kg body weight/day, 20 mg/kg body weight/day, 25 mg/kg body weight/day, 30 mg/kg body weight/day, 35 mg/kg body weight/day, 40 mg/kg body weight/day, 45 mg/kg body weight/day, or 50 mg/kg body weight/day. As an example, when the patient is a human, the dose administered to the patient can be 57 mg/70 kg to 171 mg/70 kg.

The composition administered to the patient can be in the form of the pharmaceutical composition described above. These compositions can be sterilized by conventional sterilization techniques or by filter sterilization. The aqueous solutions can be packaged for use as is, or lyophilized and the lyophilized formulation is mixed with a sterile aqueous carrier prior to administration. The pH of the compound formulation is usually 3-11, more preferably 5-9, and most preferably 7-8. It will be understood that the use of certain excipients, carriers, or stabilizers described above can result in the formation of a pharmaceutical salt.

The present disclosure provides use of at least one of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof in analytical assays for identifying a compound capable of inhibiting one or more kinases, wherein the kinase is preferably LRRK, more preferably LRRK2.

Preferably, the analytical assay is a competitive binding activity assay.

More preferably, the competitive binding assay comprises contacting the compound of the present disclosure with a kinase and detecting any change in the interaction between the compound according to the present disclosure and the kinase.

Another aspect of the present disclosure provides a method for detecting the binding of the compound of the present disclosure to a kinase, the method comprising the following steps:

(i) contacting at least one of the compound of the present disclosure, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof with a kinase in the presence of the kinase and a known substrate; and
(ii) detecting any change in the interaction between the kinase and the known substrate.

**Beneficial Effects**

The compound provided by the present disclosure has good modulating/inhibition effects on LRRK2 kinase, and can be used for treating disorders and diseases associated with LRRK2 kinase activity and for manufacturing a medicament for such disorders and diseases. The compound of the present disclosure has simple preparation method and good application prospect.

**Definitions and Description of terms**

Unless otherwise stated, the definitions of groups and terms described in the specification and claims of the present application, including definitions thereof as examples, exemplary definitions, preferred definitions, definitions documented in tables, definitions of specific compounds in the examples, and the like, can be arbitrarily combined and incorporated with each other. The definitions of groups and the structures of the compounds in such combinations and incorporations should be construed as being within the scope of the specification and/or the claims of the present application.

Unless otherwise stated, a numerical range set forth in the specification and claims shall be construed as at least including each specific integer value within the range. For example, the numerical range of "1-10" shall be construed as including each integer value in the numerical range "1-10", i.e., 1, 2, 3, 4, 5, 6, 7, 8, 9, and 10. Moreover, when certain numerical ranges are defined as "a number", it shall be construed as including both endpoints of the range, each integer within the range, and each decimal within the range. For example, "a number of 0-10" shall be construed as including not only each of integers 0, 1, 2, 3, 4, 5, 6, 7, 8, 9, and 10, but also at least the sums of each integer and 0.1, 0.2, 0.3, 0.4, 0.5, 0.6, 0.7, 0.8, or 0.9.

It should be understood that when one, two, or more are described herein, "more" shall mean an integer greater than 2, e.g., an integer greater than or equal to 3, e.g., 3, 4, 5, 6, 7, 8, 9, or 10.

The term "halogen" refers to fluorine, chlorine, bromine, and iodine.

The term "$C_{1-40}$ alkyl" should be understood that it refers to a linear or branched saturated monovalent hydrocarbyl group having 1-40 carbon atoms. For example, "$C_{1-10}$ alkyl" refers to a linear or branched alkyl group having 1, 2, 3, 4, 5, 6, 7, 8, 9, or 10 carbon atoms and "$C_{1-6}$ alkyl" refers to a linear or branched alkyl group having 1, 2, 3, 4, 5, or 6 carbon atoms. The alkyl is, for example, methyl, ethyl, n-propyl, n-butyl, n-pentyl, n-hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof.

The term "$C_{3-10}$ cycloalkyl" should be understood that it refers to a saturated monovalent monocyclic or bicyclic (e.g., bridged ring or spiro ring) hydrocarbon ring or tricyclic alkane having 3, 4, 5, 6, 7, 8, 9, or 10 carbon atoms. The $C_{3-10}$ cycloalkyl can be monocyclic hydrocarbyl such as cyclopropyl, cyclobutyl, cyclopentyl, cyclohexyl, cycloheptyl, cyclooctyl, cyclononyl or cyclodecyl, or bicyclic hydrocarbyl such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, 2,6-diazaspiro[3,4]octyl, or tricyclic hydrocarbyl such as adamantyl.

Unless otherwise defined, the term "3- to 10-membered heterocyclyl" refers to a saturated or unsaturated non-aromatic ring or ring system; for example, it is a 4-, 5-, 6- or 7-membered monocyclic ring system, a 7-, 8-, 9-, or 10-membered bicyclic (e.g., fused ring, bridged ring, or spiro ring) ring system, or a 10-membered tricyclic ring system, and comprises at least one, e.g., 1, 2, 3, 4, 5 or more heteroatoms selected from O, S, and N, wherein N and S can also be optionally oxidized to various oxidized forms to form nitrogen oxides, -S(O)-, or -$S(O)_2$-. Preferably, the heterocyclyl can be selected from "3- to 10-membered heterocyclyl". The heterocyclyl can be attached to the rest of the molecule through any one of the carbon atoms or the nitrogen atom(s) (if present). The heterocyclyl may include fused or bridged rings as well as spiro rings. In particular, the heterocyclyl may include, but is not limited to: 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl and trithianyl; or 7-membered rings such as diazepanyl. Optionally, the heterocyclyl can be benzo-fused. The heterocyclyl can be bicyclic, for example, but not limited to, a 5,5-membered ring such as a hexahydrocyclopenta[c]pyrrol-2(1H)-yl ring, or a 5,6-membered bicyclic ring such as a hexahydropyrrolo[1,2-*a*]pyrazin-2(1*H*)-yl ring. Heterocyclyl can be partially unsaturated, i.e., it can contain one or more double bonds, for example, but not limited to, dihydrofuranyl, dihydropyranyl, 2,5-dihydro-1H-pyrrolyl, 4H-[1,3,4]thiadiazinyl, 4,5-dihydrooxazolyl or 4H-[1,4]thiazinyl, or it can be benzo-fused, for example, but not limited to, dihydroisoquinolyl. When the 3- to 10-membered heterocyclyl is attached to another group to form the compound of the present disclosure, the group may be attached to the carbon atom on the 3- to 10-membered heterocyclyl, or may be attached to the heteroatom on the 3- to 10-membered heterocyclyl. For example, when the 3- to 10-membered heterocyclyl is piperazinyl, the group may be attached to the nitrogen atom on the piperazinyl. Alternatively, when the 3- to 10-membered heterocyclyl is piperidyl, the group may be attached to the nitrogen atom on the piperidyl ring or the carbon atom in the para position.

The term "$C_{6-10}$ aryl" should be understood that it preferably refers to an aromatic or partially aromatic monovalent monocyclic or bicyclic (e.g., fused ring, bridged ring, or spiro ring) hydrocarbon ring having 6-10 carbon atoms, and can be a single aromatic ring or multiple aromatic rings fused together, in particular a ring having 6 carbon atoms ("$C_6$ aryl"), e.g., phenyl or biphenyl, or a ring having 9 carbon atoms ("$C_9$ aryl"), e.g., indanyl or indenyl, or a ring having 10 carbon atoms ("$C_{10}$ aryl"), e.g., naphthyl. When the $C_{6-10}$ aryl is substituted, it can be monosubstituted or polysubstituted. In addition, the substitution site is not limited, and can be, for example, in ortho-, para-, or meta-position.

The term "5- to 10-membered heteroaryl" should be understood that it includes a monovalent monocyclic, bicyclic (e.g., fused ring, bridged ring, or spiro ring), or tricyclic aromatic ring system, which has 5-10 ring atoms and comprises 1-5 heteroatoms independently selected from N, O, and S, preferably 1-3 heteroatoms each independently selected from N, O, and S, and can be benzo-fused in each case. "Heteroaryl" also refers to a group in which a heteroaromatic ring is fused to one or more aryl, alicyclic or heterocyclyl rings, wherein the radical or site of attachment is on the heteroaromatic ring. Non-limiting examples of heteroaryl include 1-, 2-, 3-, 5-, 6-, 7- or 8-indolizinyl, 1-, 3-, 4-, 5-, 6- or 7-isoindolyl, 2-, 3-, 4-, 5-, 6- or 7-indolyl, 2-, 3-, 4-, 5-, 6- or 7-indazolyl, 2-, 4-, 5-, 6-, 7- or 8-purinyl, 1-, 2-, 3-, 4-, 6-, 7-, 8- or 9-quinolizinyl, 2-, 3-, 4-, 5-, 6-, 7- or 8-quinolyl, 1-, 3-, 4-, 5-, 6-, 7- or 8-isoquinolyl, 1-, 4-, 5-, 6-, 7- or 8-phthalazinyl, 2-, 3-, 4-, 5- or 6-naphthyridinyl, 2-, 3-, 5-, 6-, 7- or 8-quinazolinyl, 3-, 4-, 5-, 6-, 7- or 8-cinnolinyl, 2-, 4-, 6- or 7-pteridinyl, 1-, 2-, 3-, 4-, 5-, 6-, 7- or 8-4aH-carbazolyl, 1-, 2-, 3-, 4-, 5-, 6-, 7- or 8-carbazolyl, 1-, 3-, 4-, 5-, 6-, 7-, 8- or 9-carbolinyl, 1-, 2-, 3-, 4-, 6-, 7-, 8-, 9- or 10-phenanthridinyl, 1-, 2-, 3-, 4-, 5-, 6-, 7-, 8- or 9-acridinyl, 1-, 2-, 4-, 5-, 6-, 7-, 8- or 9-perimidinyl, 2-, 3-, 4-, 5-, 6-, 8-, 9- or 10-phenanthrolinyl, 1-, 2-, 3-, 4-, 6-, 7-, 8- or 9-phenazinyl, 1-, 2-, 3-, 4-, 6-, 7-, 8-, 9- or 10-phenothiazinyl, 1-, 2-, 3-, 4-, 6-, 7-, 8-, 9- or 10-phenazinyl, 2-, 3-, 4-, 5-, 6- or 1-, 3-, 4-, 5-, 6-, 7-, 8-, 9- or 10-benzisoquinolyl, 2-, 3-, 4- or thieno[2,3-b] furanyl, 2-, 3-, 5-, 6-, 7-, 8-, 9-, 10- or 11-7*H*-pyrazino[2,3-*c*]carbazolyl, 2-, 3-, 5-, 6- or 7-2*H*-furo[3,2-*b*]-pyranyl, 2-, 3-, 4-, 5-, 7- or 8-5H-pyrido[2,3-*d*]-o-oxazinyl, 1-, 3- or 5-1H-pyrazolo[4,3-d]-oxazolyl, 2-, 4- or 5-4H-imidazo[4,5-d]thiazolyl, 3-, 5- or 8-pyrazino[2,3-d]pyridazinyl, 2-, 3-, 5- or 6-imidazo[2,1-b]thiazolyl, 1-, 3-, 6-, 7-, 8- or 9-furo[3,4-c]cinnolinyl, 1-, 2-, 3-, 4-, 5-, 6-, 8-, 9-, 10- or 11-4H-pyrido[2,3-c]carbazolyl, 2-, 3-, 6- or 7-imidazo[1,2-b][1,2,4]triazinyl, 7-benzo[b]thienyl, 2-, 4-, 5-, 6- or 7-benzoxazolyl, 2-, 4-, 5-, 6- or 7-benzimidazolyl, 2-, 4-, 4-, 5-, 6- or 7-benzothiazolyl, 1-, 2-, 4-, 5-, 6-, 7-, 8- or 9-benzoxapinyl, 2-, 4-, 5-, 6-, 7- or 8-benzoxazinyl, and 1-, 2-, 3-, 5-, 6-, 7-, 8-, 9-, 10- or 11-1*H*-pyrrolo[1,2-b][2]benzazapinyl. Typical fused heteroaryl groups include, but are not limited to, 2-, 3-, 4-, 5-, 6-, 7- or 8-quinolyl, 1-, 3-, 4-, 5-, 6-, 7- or 8-isoquinolyl, 2-, 3-, 4-, 5-, 6- or 7-indolyl, 2-, 3-, 4-, 5-, 6- or 7-benzo[b]thienyl, 2-, 4-, 5-, 6- or 7-benzoxazolyl, 2-, 4-, 5-, 6- or 7-benzimidazolyl and 2-, 4-, 5-, 6- or 7-benzothiazolyl. When the 5-to 10-membered heteroaryl is attached to another group to form the compound of the present disclosure, the group can be attached to the carbon atom on the 5- to 10-membered heteroaryl ring, or can be attached to the heteroatom on the 5- to 10-membered heteroaryl ring. When the 5- to 10-membered heteroaryl is substituted, it may be monosubstituted or polysubstituted. In addition, the substitution site is not limited. For example, hydrogen attached to the carbon atom on the heteroaryl ring can be substituted, or hydrogen attached to the heteroatom on the heteroaryl ring can be substituted.

The term "spiro ring" refers to a ring system in which two rings share 1 ring-forming atom.

The term "fused ring" refers to a ring system in which two rings share 2 ring-forming atoms.

The term "bridged ring" refers to a ring system in which two rings share not less than 3 ring-forming atoms.

Unless otherwise stated, the heterocyclyl, heteroaryl, and heteroarylene include all possible isomeric forms thereof, e.g., position isomers thereof. Thus, for some illustrative non-limiting examples, forms involving substitutions at or

bonding to other groups at one, two or more of positions 1, 2, 3, 4, 5, 6, 7, 8, 9, 10, and the like (if present) are included, including pyridin-2-yl, pyridinylene-2-yl, pyridin-3-yl, pyridinylene-3-yl, pyridin-4-yl and pyridinylene-4-yl; thienyl or thienylene, including thien-2-yl, thien-2-ylene, thien-3-yl, and thien-3-ylene; pyrazol-1-yl, pyrazol-3-yl, pyrazol-4-yl and pyrazol-5-yl.

The term "oxo" refers to that the carbon atom, nitrogen atom, or sulfur atom in the substituent is substituted with an oxy group formed after oxidation (=O).

Unless otherwise stated, the definitions of the terms used herein are also applicable to groups comprising the terms. For example, the definition of $C_{1-10}$ alkyl is also applicable to $C_{1-10}$ alkyloxy, $C_{3-10}$ cycloalkyloxy, and the like.

It will be understood by those skilled in the art that the compound represented by formula (I) can be present in the form of various pharmaceutically acceptable salts. If such compounds have basic centers, they can form acid addition salts; if such compounds have acidic centers, they can form base addition salts; if these compounds comprise both acidic centers (e.g., carboxyl) and basic centers (e.g., amino), they can also form internal salts.

The compound of the present disclosure can be present in the form of a solvate (e.g., hydrate), and the compound of the present disclosure comprises a polar solvent as a structural element of the crystal lattice of the compound, particularly, for example, water, methanol, or ethanol. The amount of the polar solvent, especially water, can be present in a stoichiometric or non-stoichiometric ratio. According to the molecular structure, the compound of the present disclosure can be chiral and may therefore be present in various enantiomeric forms. These compounds may therefore be present in a racemic or optically active form. The compound of the present disclosure encompasses isomers with each chiral carbon in R or S configuration, or mixtures and racemates thereof. The compound of the present disclosure or the intermediate thereof may be separated into enantiomers by chemical or physical methods well known to those skilled in the art, or used in such form for synthesis. In the case of racemic amines, diastereoisomers are prepared from mixtures by reaction with optically active resolving agents. Examples of suitable resolving agents are optically active acids such as R- or S-tartaric acid, diacetyltartaric acid, dibenzoyltartaric acid, mandelic acid, malic acid, lactic acid, suitable N-protected amino acids (e.g., N-benzoylproline or N-benzenesulfonylproline), or various optically active camphorsulfonic acids. Enantiomeric resolution by chromatography can be advantageously conducted with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvents containing water or alcohol, for example, hexane/isopropanol/acetonitrile.

The corresponding stable isomers can be separated according to known methods, such as extraction, filtration, or column chromatography.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that causes a biological or medical response that researchers, veterinarians, physicians or other clinicians are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition (i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms). The therapeutically effective amount can be estimated initially from cell culture assays, or the initial dose can be estimated from *in-vivo* data. Using these preliminary guidance, those of ordinary skill in the art can determine effective dosages for humans. In addition, the toxicity and the therapeutic efficacy of the compounds described herein can also be determined by standard pharmaceutical procedures in cell cultures or experimental animals, e.g., by the measurement of $LD_{50}$ and $ED_{50}$.

The term "diseases or disorders associated with LRRK kinase or LRRK2 kinase activity" refers to a disease or disorder characterized by inappropriate such kinase activity or overactivity of a kinase, as defined herein. Inappropriate activity refers to (i) kinase expression in cells that generally do not express the kinase; (ii) increased kinase expression, resulting in undesired cell proliferation, differentiation and/or growth; or (iii) reduced kinase expression, resulting in an undesirable reduction in cell proliferation, differentiation and/or growth. The overactivity of a kinase refers to the amplification of a gene encoding a particular kinase or the generation of a kinase activity level that may be associated with a disorder of cell proliferation, differentiation and/or growth (i.e., as the level of kinase increases, the severity of one or more symptoms of the cellular disorder increases). The overactivity may also be the result of unrelated or constitutive activation of the ligand due to mutations such as deletion of a fragment of the kinase responsible for ligand binding.

Examples of "auxiliary materials" as described herein are found in "Handbook of Pharmaceutical Excipients, 2nd edition, 1994, edited by A Wade and PJ Weller".

The "carriers" or "diluents" described herein are well known in the pharmaceutical art and are described, for

example, in Remington's Pharmaceutical Sciences, Mack Publishing Co. (A.R. Gennaro, edited in 1985).

The pharmaceutical carriers, auxiliary materials, or diluents can be selected with regard to the intended route of administration and standard pharmaceutical practice. The pharmaceutical composition may contain, or additionally contain, any suitable adhesives, lubricants, suspending agents, coating agents, solubilizers, buffers, flavoring agents, surfactants, thickeners, preservatives (including antioxidants), and the like as carriers, auxiliary materials or diluents, as well as substances included to render the formulation isotonic with the blood of the recipient.

Pharmaceutical formulations suitable for oral administration with the carrier being a solid are most preferably in the form of unit dose formulations such as pills, capsules or tablets each containing a predetermined amount of the active compound. Tablets can be prepared by compression or molding, optionally together with one or more additional ingredients. Compressed tablets can be prepared by compressing in a suitable machine the active compound in a free-flowing form (such as powders or granules), optionally mixed with an adhesive, a lubricant, an inert diluent, a lubricating substance, a surfactant or a dispersing agent. Molded tablets can be prepared by molding the active compound with an inert liquid diluent. The tablets may optionally be coated and if not, symbols may optionally be printed thereon. Capsules can be prepared by filling capsule shells with the active compound alone or in admixture with one or more additional ingredients and then sealing in a conventional manner. Cachets are similar to capsules in that the active compound is enclosed in rice paper film along with any additional ingredients. The active compound may also be formulated into dispersible granules, so as to, for example, suspend it in water or spread it on food before administration. The granules can be packaged in, for example, a sachet. The carriers may be liquid formulations suitable for oral administration, and may be presented in the form of a solution or suspension in an aqueous or non-aqueous liquid, or in the form of an oil-in-water liquid emulsion.

The term "pharmaceutically acceptable salt" includes suitable acid addition salts or base salts thereof. Suitable pharmaceutical salts are described in J Pharm Sci., 66, 199, 1977, Berge et al. For example, salts are formed with strong inorganic acids, such as mineral acids, for example hydrohalic acids (e.g. hydrochloric acid, hydrobromic acid, and hydroiodic acid), sulfuric acid, phosphoric acid sulfate, hydrosulfate, hemisulfate, thiocyanate, persulfate, and sulfonic acid; with strong organic carboxylic acids, such as unsubstituted or substituted (e.g., halogen-substituted) alkanecarboxylic acids of 1 to 4 carbon atoms, such as acetic acid; with saturated or unsaturated dicarboxylic acids, such as oxalic acid, malonic acid, succinic acid, maleic acid, fumaric acid, phthalic acid or tetraphthalic acid; with hydroxycarboxylic acids, such as ascorbic acid, glycolic acid, lactic acid, malic acid, tartaric acid or citric acid; with amino acids, such as aspartic acid or glutamic acid; with benzoic acid; or with organic sulfonic acids, such as unsubstituted or substituted (e.g., halogen-substituted) ($C_1$-$C_4$)-alkyl- or aryl-sulfonic acid, such as methane-, or *p*-toluenesulfonic acid.

Preferred salts include, for example, acetate, trifluoroacetate, lactate, gluconate, citrate, tartrate, maleate, malate, pantothenate, adipate, alginate, aspartate, benzoate, butyrate, digluconate, cyclovalerate, glucoheptonate, glycerophosphate, oxalate, heptanoate, hexanoate, fumarate, nicotinate, palmitate, pectate, 3-phenylpropionate, picrate, pivalate, propionate, tartrate, lactobionate, pivolate, camphorate, undecanoate, and succinate; salts of organic sulfonic acids such as methanesulfonate, ethanesulfonate, 2-hydroxyethanesulfonate, camphorsulfonate, 2-naphthalenesulfonate, benzenesulfonate, *p*-chlorobenzenesulfonate, and *p*-toluenesulfonate; and salts of inorganic acids such as hydrochloric acid, hydrobromic acid, hydroiodic acid, sulfuric acid, hydrogen sulfate, hemisulfuric acid, thiocyanic acid, persulfuric acid, phosphoric acid, and sulfonic acid.

The term "pharmaceutically acceptable ester" refers to an ester formed by a functional group that may be esterified in the structure of the compound of the present disclosure with an organic acid or alcohol/hydroxide. Organic acids include carboxylic acids, such as unsubstituted or substituted (e.g., halogen-substituted) alkanecarboxylic acids of 1 to 12 carbon atoms, such as acetic acid; saturated or unsaturated dicarboxylic acids, such as oxalic acid, malonic acid, succinic acid, maleic acid, fumaric acid, phthalic acid or tetraphthalic acid; hydroxycarboxylic acids, such as ascorbic acid, glycolic acid, lactic acid, malic acid, tartaric acid or citric acid; amino acids, such as aspartic acid or glutamic acid; benzoic acid; or organic sulfonic acids, such as unsubstituted or substituted (e.g., halogen-substituted) ($C_1$-$C_4$)-alkyl- or aryl-sulfonic acid, such as methane-, or *p*-toluenesulfonic acid. Suitable hydroxides include inorganic hydroxides such as sodium hydroxide, potassium hydroxide, calcium hydroxide, and aluminum hydroxide. Alcohols include alkanols of 1-12 carbon atoms that may be unsubstituted or substituted (e.g., with halogen).

The term "isotopically labeled compound" means that at least one atom in the compound of the present disclosure is replaced with an isotope. Examples of the isotopes include isotopes of hydrogen, carbon, nitrogen, oxygen, phosphorus, sulfur, fluorine, and chlorine, such as the corresponding $^{2}H$, $^{3}H$, $^{13}C$, $^{14}C$, $^{15}N$, $^{17}O$, $^{18}O$, $^{31}P$, $^{32}P$, $^{35}S$, $^{18}F$, and $^{36}Cl$. Substitution with isotopes such as deuterium (i.e., $^{2}H$) may afford certain therapeutic advantages (i.e., increased *in-vivo* half-life or reduced dose requirements) resulting from greater metabolic stability and hence may be preferred in some circumstances. For example, the present disclosure comprises the compound represented by general formula (I) wherein any hydrogen atom is replaced with a deuterium atom.

The term "prodrug compound" refers to a covalently-bonded compound that can release the active parent drug according to general formula (I) *in vivo*. Such prodrug is generally the compound of the present disclosure wherein one or more appropriate groups have been modified such that, upon administration to a human or mammalian subject, the

modification may be reversed. The reversion is typically performed by an enzyme naturally present in such subjects, although it is possible to administer a second medicament with such the prodrug in order to allow for reversion *in vivo.* Examples of such modifications include pharmaceutically acceptable esters as described above, wherein such reversion may be performed via esterases and the like.

The term "polymorph" refers to the compound of the present disclosure in various forms of crystallines, polymorphs, and hydrates. It is well established in the pharmaceutical industry that chemical compounds can be isolated in any of these forms by methods of purification and/or separation of the solvents used in the synthetic manufacturing of such compounds.

The term "administration" means that the pharmaceutical composition of the present disclosure may be suitable for rectal, intranasal, intrabronchial, topical (including buccal and sublingual), vaginal or parenteral (including subcutaneous, intramuscular, intravenous, intraarterial, and intradermal), intraperitoneal or intrathecal administration. Preferably, the formulation is a formulation for oral administration. The formulations may conveniently be presented in unit dosage form, i.e., in the form of discrete parts containing a unit dose or a plurality of units or subunits of a unit dose. As an example, the formulation may be in the form of tablets and sustained release capsules, and may be prepared by any of the methods well known in the pharmaceutical art.

The formulation for oral administration of the present disclosure may be presented as: discrete units such as capsules, gels, drops, cachets, pills, or tablets each containing a predetermined amount of the active agent; as powders or granules; as a solution, emulsion or suspension of the active agent in an aqueous or non-aqueous liquid; or as an oil-in-water liquid emulsion or a water-in-oil liquid emulsion; or as a bolus preparation, and the like. Preferably, these compositions contain 1-250 mg and more preferably 10-100 mg of active ingredient per dose.

For compositions for oral administration (e.g., tablets and capsules), solvents and/or conventional auxiliary materials are also included, such as adhesives, for example, syrup, acacia, gelatin, sorbitol, tragacanth, polyvinylpyrrolidone (povidone), methylcellulose, ethylcellulose, sodium carboxymethylcellulose, hydroxypropylmethylcellulose, sucrose, and starch; fillers and carriers, for example, corn starch, gelatin, lactose, sucrose, microcrystalline cellulose, kaolin, mannitol, dicalcium phosphate, sodium chloride, and alginic acid; and lubricants, for example, magnesium stearate, sodium stearate and other metal stearates, glyceryl stearate, stearic acid, silicone fluids, talc, waxes, oils, and colloidal silica. Flavoring agents such as peppermint, wintergreen oil, cherry flavoring, and the like can also be used. It may be desirable to add a colorant to make the dosage form easily identifiable. Tablets may also be coated by methods well known in the art.

Other formulations suitable for oral administration include lozenges containing the active agent in a flavored matrix, typically sucrose and acacia or tragacanth; pastilles containing the active agent in an inert matrix such as gelatin and glycerin, or sucrose and acacia; and mouthwashes containing the active agent in a suitable liquid carrier.

Other forms of administration include solutions or emulsions prepared from sterile or sterilizable solutions for intravenous, intraarterial, intrathecal, subcutaneous, intradermal, intraperitoneal, or intramuscular injection. Injectable forms generally contain 10-1000 mg, preferably 10-250 mg, of active ingredient per dose.

The forms of administration may also be a combination administration, i.e., one or more of the compounds of the present disclosure are administered in combination with one or more of other active agents. In such cases, the compound of the present disclosure may be administered with one or more of other active agents consecutively, simultaneously, or sequentially.

**Analytical assay**

Another aspect of the present disclosure relates to use of the compound as described above in an analytical assay to identify the capacity of other candidate compounds to inhibit one or more kinases, more preferably LRRK, and even more preferably LRRK2.

Preferably, the analytical assay is a competitive binding assay.

More preferably, the competitive binding assay comprises contacting the compound of the present disclosure with a kinase, preferably LRRK, and more preferably LRRK2 and a candidate compound, and detecting any change in the interaction between the compound according to the present disclosure and the kinase.

Preferably, the candidate compound is generated by SAR modification of the compound of the present disclosure.

As used herein, the term "SAR modification" refers to a standard method of altering a given compound by chemical derivatization.

Thus, in one aspect, the identified compound can be used as a model (e.g., template) for the development of other compounds. The compounds used in such assays may be free in solution, fixed onto a solid carrier, supported on the surface of cells, or located in the cells. The elimination of activity or the formation of a binding complex between a compound and a medicament to be tested can be measured.

The analytical assay of the present disclosure can be a screening, whereby a large number of medicaments are

tested. In one aspect, the analytical assay method of the present disclosure is a high-throughput screening.

The present disclosure also contemplates the use of competitive drug screening assays in which neutralizing antibodies capable of binding to a compound specifically compete with a test compound for binding to the compound.

Another technique for screening is provided for high-throughput screening (HTS) of agents with suitable binding affinity for a substance and is based on the method described in detail in WO 84/03564.

It is contemplated that the analytical assay method of the present disclosure will be suitable for small-scale and large-scale screening of test compounds as well as for quantitative assays.

Preferably, the competitive binding assay comprises contacting the compound of the present disclosure with a kinase in the presence of a known substrate for the kinase, and detecting any change in the interaction between the kinase and the known substrate.

Another aspect of the present disclosure provides a method for detecting the binding of a ligand to a kinase, the method comprising the following steps:

(i) contacting a ligand with a kinase in the presence of a known substrate for the kinase; and
(ii) detecting any change in the interaction between the kinase and the known substrate, wherein the ligand is the compound of the present disclosure.

One aspect of the present disclosure relates to a method comprising the following steps:

(a) performing the assay method described above;
(b) identifying one or more ligands capable of binding to a ligand binding domain;
(c) modifying the one or more ligands capable of binding to the ligand binding domain;
(d) performing the assay method described above; and
(e) optionally preparing a pharmaceutical composition comprising the one or more ligands.

The present disclosure further relates to a ligand identified by the method described above. Another aspect of the present disclosure relates to a pharmaceutical composition comprising a ligand identified by the method described above.

Another aspect of the present disclosure relates to use of a ligand identified by the method described above for manufacturing a pharmaceutical composition for the treatment of one or more disorders. The method described above can be used for screening for ligands that may be used as inhibitors of one or more kinases.

## BRIEF DESCRIPTION OF THE DRAWINGS

FIG. 1 is an exemplary graph showing the results of western blot analysis for the LRRK2 expression level in the brain tissues of LRRK2-G2019S mice in Example 12.

FIG. 2 shows the effect of compound 527 on the phosphorylation level of LRRK2 in the brain tissues of LRRK2-G2019S mice in Example 12. Data are presented as mean $\pm$ sample standard deviation (mean $\pm$ SE); n = 8; * indicates $p < 0.05$ compared to the vehicle control group, and *** indicates $p < 0.001$ compared to the vehicle control group, as determined by one-way analysis of variance.

## DETAILED DESCRIPTION

The technical solutions of the present disclosure will be further explained in detail by the description of the following specific examples. It should be understood that the following examples are merely exemplary illustrations and explanations of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared by using known methods.

The abbreviations in the context of the present disclosure have the following meanings:

| | |
|---|---|
| DBU | 1,8-diazabicyclo[5.4.0]undec-7-ene |
| DCM | dichloromethane |
| DEA | diethylamine |
| DIEA | diisopropyl ethyl amine |
| DMA | *N*,*N*-dimethylacetamide |

| | |
|---|---|
| DMF | *N,N*-dimethylformamide |
| DMSO | dimethylsulfoxide |
| EA, EtOAc | ethyl acetate |
| IPA | isopropanol |
| MTBE | *tert*-butyl methyl ether |
| NIS | *N*-iodosuccinimide |
| NMR | nuclear magnetic resonance |
| PE | petroleum ether |
| PMBCl | *p*-methoxybenzyl chloride |
| RT, $t_R$ | retention time |
| SFC | supercritical fluid chromatography |
| Tf | trifluoromethanesulfonyl |
| TfOH | trifluoromethanesulfonic acid |
| TFA | trifluoroacetic acid |
| THF | tetrahydrofuran |
| UV | ultraviolet ray |

**Chromatography**

High pressure liquid chromatography is performed and monitored by a multi-wavelength UV detector. Typical mobile phases for the separation process are PE/EA, DCM/MeOH, or water/MeCN.

**Analysis Procedures**

$^1$H nuclear magnetic resonance (NMR) spectroscopy is performed at room temperature in the solvent using Bruker AV 400 spectrometer unless otherwise stated. In all cases, the NMR data are consistent with the proposed structure. Using conventional abbreviations for designating the main peaks, the characteristic chemical shifts (δ) are given in parts per million: for example, s, singlet; d, doublet; t, triplet; q, quartet; dd, doublet of doublets; br, broad. Mass spectra are recorded using Agilent 1290 Infinity/6460 triple Quad LCMS. When thin-layer chromatography (TLC) is used, it refers to silica gel TLC.

**Preparation of Compounds**

Without the description of the preparation of starting materials, these starting materials may be purchased commercially, known in the literature, or readily available to those skilled in the art using standard procedures. In the case where compounds are illustrated as being prepared through analogy to previous examples or intermediates, it will be appreciated by those skilled in the art that the reaction time, number of equivalents of reagents, and temperature for each particular reaction can be varied, and that it may be necessary or desirable to employ different post-treatment or purification techniques.

**Example 1: Preparation of Compound 390**

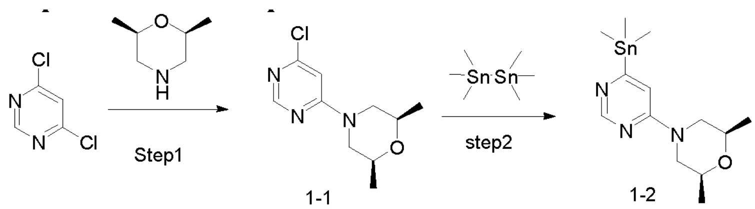

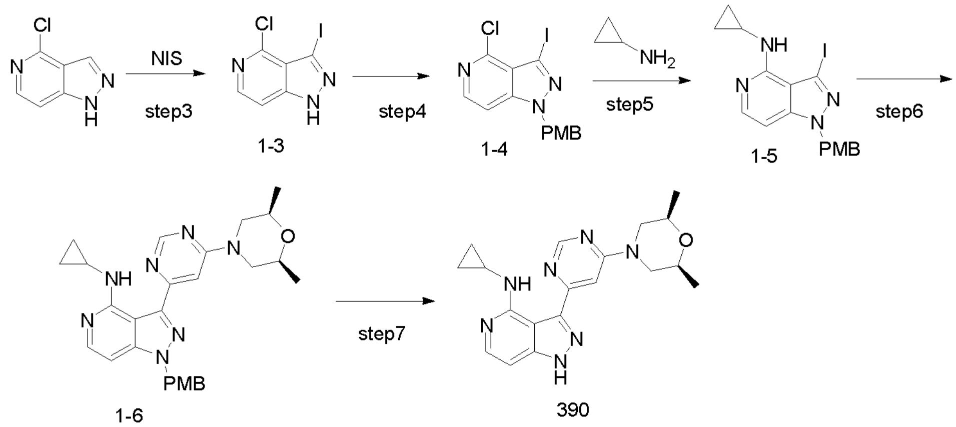


1-3 1-4 1-5 1-6 390

**Step 1: Synthesis of (2*S*,6*R*)-4-(6-chloropyrimidin-4-yl)-2,6-dimethylmorpholine (1-1)**

DIEA (87.7 g, 0.680 mol) and (2*R*,6*S*)-2,6-dimethylmorpholine (40.5 g, 0.350 mol) were added to a solution of 4,6-dichloropyrimidine (50.0 g, 0.340 mol) in IPA (1000 mL) at room temperature. The mixture was stirred at room temperature for 1 h, and concentrated to remove the solvent. The residue was diluted in EtOAc (500 mL). The organic layer was washed with brine, dried over anhydrous $Na_2SO_4$, filtered, and concentrated to give a residue. The residue was purified by silica gel column chromatography (PE/EA = 1:1) to give (2S,6R)-4-(6-chloropyrimidin-4-yl)-2,6-dimethylmorpholine (1-1, 76.0 g, 98.4%) in the form of a white solid. m/z $(ESI)^+$: 228.0 $[M+H]^+$.

**Step 2: Synthesis of (2S,6R)-2,6-dimethyl-4-(6-(trimethylstannyl)pyrimidin-4-yl)morpholine (1-2)**

A dry three-necked flask containing xylene (100 mL) was heated to 150 °C under $N_2$ atmosphere, and then a degassed mixture of a solution of compound 1-1 (10.0 g, 44.1 mmol) in xylene (50 mL), 1,1,1,2,2,2-hexamethylstilbene (18.8 g, 57.3 mmol), $Pd(PPh_3)_2Cl_2$ (3.00 g, 4.40 mmol), and $PPh_3$ (2.30 g, 8.80 mmol) was added dropwise. The resulting mixture was stirred at 150 °C for 2 h. The reaction mixture was cooled to room temperature and diluted with PE, and then filtered through celite. The filtrate was concentrated under vacuum to give a crude product (1-2, 16.0 g, purity: about 60%) in the form of a yellow solid, which was used in the next step without further purification, m/z $(ESI)^+$: 358.1 $[M+H]^+$.

**Step 3: Synthesis of 4-chloro-3-iodo-1*H*-pyrazolo[4,3-*c*]pyridine (1-3)**

NIS (88.0 g, 0.390 mol) was added to a solution of 4-chloro-1*H*-pyrazolo[4,3-*c*]pyridine (50.0 g, 0.326 mol) in DMF (500 mL) at room temperature, and after the dropwise addition was completed, the mixture was heated to 80 °C and stirred for 2 h. The mixture was then concentrated to give a residue, and the residue was treated with a saturated aqueous $NaHSO_3$ solution (500 mL) while stirring for 30 min. The solid formed was collected by filtration and washed with water (2 L), and then dried under vacuum to give a product (1-3, 83.0 g, 91.2%) in the form of a brown solid. m/z $(ESI)^+$: 279.8 $[M+H]^+$.

**Step 4: Synthesis of 4-chloro-3-iodo-1-(4-methoxybenzyl)-1*H*-pyrazolo[4,3-*c*]pyridine (1-4)**

PMBCl (49.6 g, 0.320 mol) and NaOH (23.2 g, 0.580 mol) were added to a solution of compound 1-3 (80.0 g, 0.290 mol) in DMF (800 mL) in an ice-water bath. The mixture was stirred at room temperature for 20 h, and concentrated to remove the solvent. The residue was dissolved in EtOAc (500 mL), washed with brine, dried over anhydrous $Na_2SO_4$, filtered and concentrated to give a residue. The residue was slurried with PE and EA (PE/EA = 1:1), stirred overnight and filtered. The solid was dried to give compound 1-4 (104 g, 89.7%) in the form of an off-white solid. m/z $(ESI)^+$: 399.7 $[M+H]^+$.

**Step 5: Synthesis of *N*-cyclopropyl-3-iodo-1-(4-methoxybenzyl)-1*H*-pyrazolo[4,3-*c*]pyridine-4-amine (1-5)**

A mixture of compound 1-4 (7.00 g, 17.5 mmol) and cyclopropylamine (10.0 g, 175 mmol) in DIEA (50 mL) was heated to 140 °C, stirred for 2 days, concentrated under vacuum to remove the solvent, and then diluted in EA and brine. The separated organic layer was dried over anhydrous $Na_2SO_4$, filtered, and concentrated to give a residue. The residue was purified by silica gel column chromatography (100% EA) to give compound 1-5 (5.00 g, 68.0%) in the form of a brown solid. m/z $(ESI)^+$: 421.1 $[M+H]^+$.

**Step 6: Synthesis of *N*-cyclopropyl-3-(6-((2S,6R)-2,6-dimethylmorpholinyl)pyrimidin-4-yl)-1-(4-methoxybenzyl)-1*H*-pyrazolo[4,3-*c*]pyridine-4-amine (1-6)**

To a mixture of compound 1-5 (200 mg, 0.476 mmol) and the crude product of compound 1-2 (purity: about 60%, 425 mg, 0.714 mmol) in DMF (10 mL), $Pd(PPh_3)_2Cl_2$ (34 mg, 0.048 mmol), $PPh_3$ (25 mg, 0.095 mmol), and CuI (9 mg, 0.043 mmol) were added under $N_2$ atmosphere. The mixture was degassed and purged with nitrogen thrice, and then stirred at 120 °C for 1 h, cooled to room temperature and diluted in EA. The organic phase was washed with a saturated aqueous KF solution and brine. The organic layer was separated, dried over anhydrous $Na_2SO_4$, filtered, and concentrated to give a residue. The residue was purified by silica gel column chromatography (DCM/MeOH = 19:1) to give compound 1-6 (50.0 mg, 21.6%) in the form of a pale brown solid. m/z $(ESI)^+$: 486.1 $[M+H]^+$.

**Step 7: Synthesis of *N*-(3,3-difluorocyclobutyl)-3-(6-(2*S*,6*R*)-2,6-dimethylmorpholinyl) pyrimidin-4-yl)-1*H*-pyrazolo[4,3-c]pyridine-4-amine (390)**

TfOH (0.5 mL) was added to a solution of compound 1-6 (50.0 mg, 0.103 mmol) in TFA (5 mL). The resulting mixture was stirred at room temperature for 1 h, concentrated to remove the solvent, diluted in DCM, and then washed with 1 M aqueous NaOH solution and brine. The organic layer was separated, dried over anhydrous $Na_2SO_4$, filtered, and concentrated to give a residue. The residue was purified by preparative TLC (DCM/MeOH = 10:1) to give compound 390 (6 mg) in the form of an off-white solid. $^1$HNMR (400 MHz, $CD_3OD$) $\delta$ 8.68 (d, $J$ = 1.0 Hz, 1H), 7.72-7.65 (m, 2H), 7.10 (d, $J$ = 7.2 Hz, 1H), 4.26-4.72 (m, 2H), 3.76-3.64 (m, 2H), 2.94-2.86 (m, 1H), 2.78-2.65 (m, 2H), 1.28 (d, $J$ = 6.2 Hz, 6H), 1.20-1.13 (m, 2H), 0.95-0.88 (m, 2H). m/z $(ESI)^+$: 366.0 $[M+H]^+$.

Referring to Example 1, the compounds shown in Table 1 were prepared using the corresponding starting materials in Step 5.

**Table 1**

| Compound | Structure | m/z $(ESI)^+$ $M+H^+$ |
|---|---|---|
| **391** | 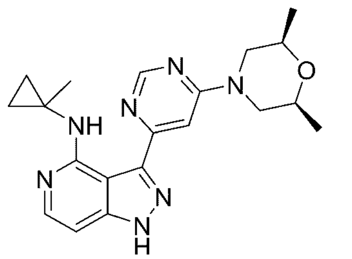 | 379.9 |
| **446** | 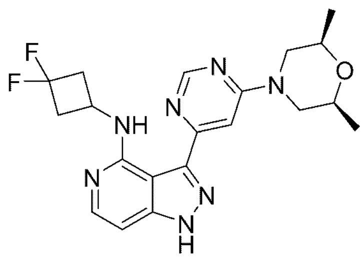 | 416.2 |
| **447** | 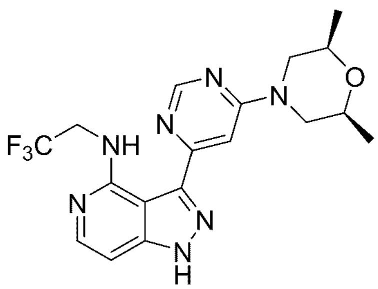 | 407.8 |

## Example 2: Preparation of Compound 397

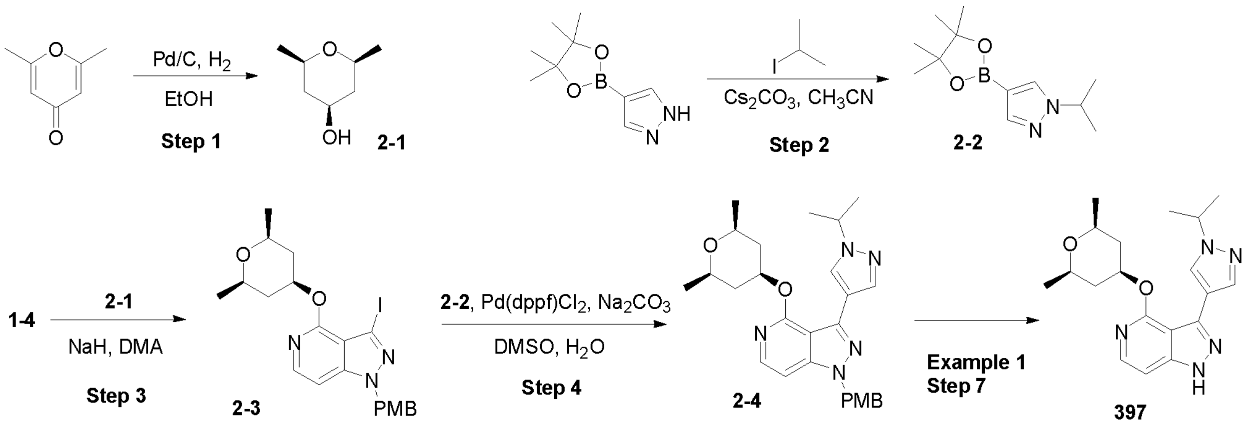


### Step 1: Synthesis of (*cis*)-2,6-dimethyltetrahydro-2*H*-pyran-4-ol (2-1)

Pd/C (10%, 0.500 g) was added to a solution of 2,6-dimethyl-4*H*-pyran-4-one (5.00 g, 40.3 mmol) in ethanol (50 mL), and the reaction mixture was stirred at 35 °C under $H_2$ (4 atm) atmosphere for 20 h. The reaction mixture was filtered through celite, and the filtrate was concentrated under reduced pressure to give compound 2-1 (5.00 g, 95.4%) in the form of a pale yellow crystal. m/z $(ESI)^+$: 131.1 $[M+H]^+$.

### Step 2: Synthesis of 1-isopropyl-4-(4,4,5,5-tetramethyl-1,3,2-dioxabenzaldehyde-2-yl)-1*H*-pyrazole (2-2)

2-Iodopropane (6.57 g, 38.6 mmol) was added to a suspension of 4-(4,4,5,5-tetramethyl-1,3,2-dioxabenzofuran-2-yl)-1*H*-pyrazole (5.00 g, 25.8 mmol) and $Cs_2CO_3$ (12.6 g, 38.6 mmol) in acetonitrile (100 mL) at room temperature. The mixture was heated to 60 °C and stirred for 20 h. The reaction mixture was filtered through celite, and the filtrate was concentrated under reduced pressure. The residue was purified by silica gel column chromatography (PE/EA = 4:1) to give compound 2-2 (5.80 g, 95.2%) in the form of a colorless oil. $^1$HNMR (300 MHz, $CDCl_3$) $\delta$ 7.78 (s, 1H), 7.73 (s, 1H), 4.51 (hept, *J* = 6.7 Hz, 1H), 1.49 (d, *J* = 6.7 Hz, 6H), 1.31 (s, 12H).

### Step 3: Synthesis of 4-((2*R*,4R,6*S*)-2,6-dimethyltetrahydro-2*H*-pyran-4-yl)oxy)-3-iodo-1-(4-methoxybenzyl)-1*H*-pyrazolo[4,3-*c*]pyridine (2-3)

NaH (60%) (187 mg, 4.68 mmol) was added to a solution of compound 2-1 (510 mg, 3.90 mmol) in DMA (10 mL) at room temperature. After the mixture was stirred for 10 min, compound 1-4 (1.57 g, 3.9 mmol) was added. The resulting mixture was heated to 120 °C and stirred for 20 h. The mixture was cooled to room temperature, and then added with water (100 mL). The resulting mixture was extracted with EtOAc (100 mL $\times$ 2). The combined organic layers were washed with brine, dried over anhydrous $Na_2SO_4$, filtered, and concentrated to give a residue. The residue was purified by silica gel column chromatography (PE/EA = 3:2) to give compound 2-3 (680 mg, 35.3%) in the form of a light yellow oil. m/z $(ESI)^+$: 493.7 $[M+H]^+$.

### Step 4: Synthesis of 4-((2*R*,4*R*,6*S*)-2,6-dimethyltetrahydro-2*H*-pyran-4-yl)oxy)-3-(1-isopropyl-1*H*-pyrazol-4-yl)-1-(4-methoxybenzyl)-1*H*-pyrazolo[4,3-*c*]pyridine (2-4)

To a mixture of compound 2-3 (176 mg, 0.357 mmol), compound 2-2 (90 mg, 0.38 mmol), and $Na_2CO_3$ (121 mg, 1.14 mmol) in DMSO/$H_2O$ (5 mL/0.5 mL), $Pd(dppf)Cl_2$ (27 mg, 0.038 mmol) was added. The mixture was degassed and purged with nitrogen thrice, and then stirred at 80 °C under nitrogen atmosphere for 20 h. The mixture was cooled to room temperature, and then added with EA and water. The organic layer was separated, washed with brine, dried over anhydrous $Na_2SO_4$, filtered, and concentrated to give a residue. The residue was purified by silica gel column chromatography (PE/EA = 32:3) to give compound 2-4 (100 mg, 59.0%) in the form of a pale brown solid. m/z $(ESI)^+$: 475.8 $[M+H]^+$.

**Step 5: Synthesis of 4-((2*R*,4*R*,6*S*)-2,6-dimethyltetrahydro-2*H*-pyran-4-yl)oxy)-3-(1-isopropyl-1*H*-pyrazol-4-yl)-1*H*-pyrazolo[4,3-c]pyridine (397)**

Similar to Step 7 in Example 1, compound 2-4 was used to synthesize 4-((2*R*,4*R*,6*S*)-2,6-dimethyltetrahydro-2*H*-pyran-4-yl)oxy)-3-(1-isopropyl-1*H*-pyrazol-4-yl)-1*H*-pyr azolo[4,3-c]pyridine (397) in the form of a white solid. $^1$HNMR (400 MHz, $CD_3OD$) $\delta$ 8.31 (s, 1H), 8.14 (s, 1H), 7.86 (d, *J* = 6.1 Hz, 1H), 7.07 (d, *J* = 6.1 Hz, 1H), 5.54-5.45 (m, 1H), 4.67-4.59 (m, 1H), 3.76-3.68 (m, 2H), 2.39-2.34 (m, 2H), 1.60 (d, *J* = 6.7 Hz, 6H), 1.47-1.38 (m, 2H), 1.28 (d, *J* = 6.2 Hz, 6H). m/z (ESI)$^+$: 355.8 [M+H]$^+$.

Referring to Example 2, the compounds shown in Table 2 were prepared using appropriate corresponding starting materials.

**Table 2**

| Compound | Structure | m/z(ESI)$^+$ [M+H]$^+$ | RT (min)[a] |
|---|---|---|---|
| **395** | 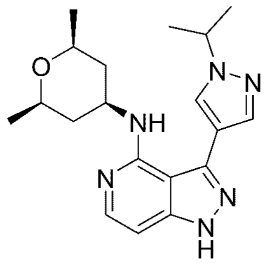 | 354.9 | 2.9 |
| **404** | 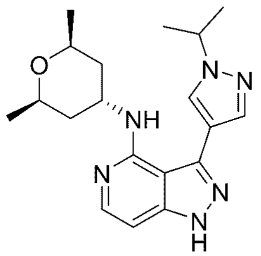 | 354.9 | 4.35 |
| **396** | 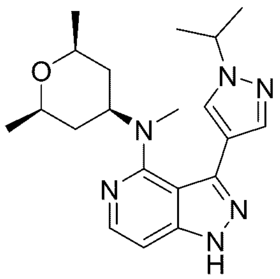 | 368.8 | 2.57 |
| **405** | 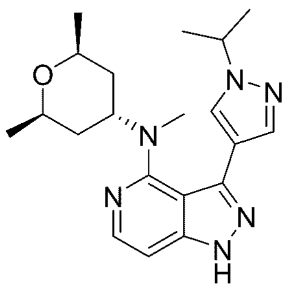 | 368.8 | 2.87 |

[a]: SFC (chiral chromatography column chiralpak-IC, 4.6 mm × 250 mm, 5 μm, eluent $CO_2$-IPA (0.2% DEA)).

**Example 3: Preparation of Compounds 407 and 421**

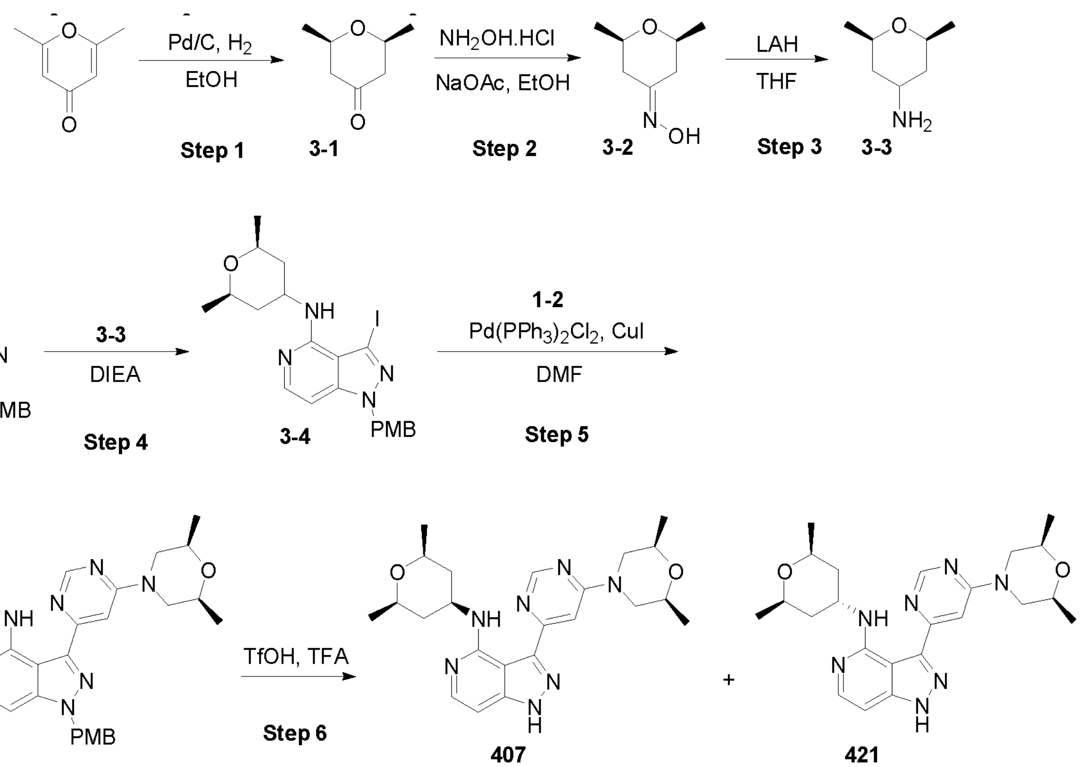


**Step 1: Synthesis of (2*R*,6*S*)-2,6-dimethyltetrahydro-4*H*-pyran-4-one (3-1)**

Pd/C (10%, 10 g) was added to a solution of 2,6-dimethyl-4*H*-pyran-4-one (100 g, 0.810 mol) in ethanol (800 mL), and the mixture was stirred at 30 °C under $H_2$ (2 atm) atmosphere for 8 h. The mixture was filtered through celite, and the filtrate was concentrated under vacuum. The residue was purified by silica gel column chromatography (PE/EA = 3:1) to give a crude product (53.0 g, 51.1%) of compound 3-1 in the form of a colorless oil. m/z $(ESI)^+$: 129.1 $[m+H]^+$.

**Step 2: Synthesis of (2*R*,6*S*)-2,6-dimethyltetrahydro-4*H*-pyran-4-one oxime (3-2)**

A suspension of compound 3-1 (15 g, 0.12 mol), hydroxylamine hydrochloride (8.1 g, 0.12 mol), and NaOAc (19.7 g, 0.24 mol) in ethanol (150 mL) was heated to 60 °C and stirred for 3 h. After cooling to room temperature, the mixture was filtered, and the filtrate was concentrated under vacuum to give a residue. The residue was diluted in EtOAc and washed with brine. The separated organic layer was dried over anhydrous $Na_2SO_4$, filtered, and concentrated to give a residue. The residue was purified by silica gel column chromatography (PE/EA = 3:7) to give compound 3-2 (16.0 g, 95.6%) in the form of a white solid. m/z $(ESI)^+$: 144.2 $[m+H]^+$.

**Step 3: Synthesis of (2*R*,6*S*)-2,6-dimethyltetrahydro-2*H*-pyran-4-amine (3-3)**

A solution of compound 3-2 (7.2 g, 0.050 mol) was added dropwise to a suspension of $LiAlH4$ (5.7 g, 0.15 mol) in THF (200 mL) at 0 °C. The mixture was then heated to 60 °C and stirred for 6 h. After cooling to 0 °C in an ice-water bath, the mixture was added sequentially with water (5.7 mL), a 15% aqueous NaOH solution (5.7 mL), and water (17.1 mL) to quench the reaction. $Mg_2SO_4$ was then added and the mixture was stirred at room temperature for 1 h. The mixture was filtered, and the filtrate was concentrated under reduced pressure to give compound 3-3 (6.10 g, 94.5%) in the form of a colorless oil. m/z $(ESI)^+$: 130.1$[m+H]^+$.

**Step 4: Synthesis of 4-((2*R*,4*R*,6*S*)-2,6-dimethyltetrahydro-2*H*-pyran-4-yl)oxy)-3-(1-isopropyl-1*H*-pyrazol-4-yl)-1-(4-methoxybenzyl)-1*H*-pyrazolo[4,3-*c*]pyridine (3-4)**

A mixture of compound 1-4 (18.6 g, 46.5 mmol) and compound 3-3 (6.00 g, 46.5 mmol) in DIEA (100 mL) was stirred at 140 °C for 36 h. The mixture was concentrated under reduced pressure to give a residue. The residue was purified by silica gel column chromatography (DCM/MeOH = 97:3) to give compound 3-4 (10.0 g, 43.7%) in the form of a brown solid. m/z $(ESI)^+$: 492.8$[m+H]^+$.

**Step 5: Synthesis of 3-(6-((2*S*,6R)-2,6-dimethylmorpholinyl)pyrimidin-4-yl)-*N*-((2*R*,6*S*)-2,6-dimethyltetrahydro-2*H*-pyran-4-yl)-1-(4-methoxybenzyl)-1*H*-pyrazolo[4,3-*c*]pyridine-4-amine (3-5)**

$Pd(PPh_3)_2Cl_2$ (280 mg, 0.400 mmol) and CuI (76 mg, 0.400 mmol) were added to a mixture of intermediate 3-4 (1.00 g, 2.00 mmol) and the crude product of intermediate 1-2 (purity: about 60%, 1.4 g, 2.35 mmol) in DMF (20 mL) under nitrogen atmosphere. The mixture was degassed and purged with nitrogen thrice, and then stirred at 120 °C for 2 h. After the mixture was cooled to room temperature and diluted with EtOAc and water, the separated organic layer was washed with brine, dried over anhydrous $Na_2SO_4$, filtered, and concentrated to give a residue. The residue was purified by silica gel column chromatography (PE/EA = 1:9) to give intermediate 3-5 (300 mg, 26.9%) in the form of a pale brown solid. m/z $(ESI)^+$: 557.7 $[m+H]^+$.

**Step 6: Synthesis of 3-(6-((2*S*,6*R*)-2,6-dimethylmorpholinyl)pyrimidin-4-yl)-N-((2*R*,4*R*,6*S*)-2,6-dimethyltetrahydro-2*H*-pyran-4-yl)-1*H*-pyrazolo[4,3-c]pyridine-4-amine (407) and 3-(6-((2*S*,6*R*)-2,6-dimethylmorpholinyl)pyrimidin-4-yl)-*N*-((2*R*,4*S*,6*S*)-2,6-dimethyltetrahydro-2*H*-pyran-4-yl)-1*H*-pyrazolo[4,3-*c*]pyridine-4-amine (421)**

TfOH (2 mL) was added to a solution of intermediate 3-5 (300 mg, 0.54 mol) in TFA (20 mL). The mixture was stirred at room temperature for 1 h. The mixture was concentrated and diluted in EtOAc, washed with 1 mol/L aqueous NaOH solution and brine, dried over anhydrous $Na_2SO_4$, filtered, and concentrated to give a residue. The residue was purified by silica gel column chromatography (PE/EA = 1:9), and then separated by preparative HPLC (water, Gemini 5 $\mu$m, C18 150 $\times$ 21.2 mm, 20 mL/min, $ACN/H_2O$ (0.1% TFA) = 33%:67%, holding 10 min) to give a white solid of compound 407 (16.5 mg) at RT = 6.55 min and a white solid of compound 421 (8.3 mg) at RT = 8.07 min.

**Compound 407:** $^1$HNMR (400 MHz, $CD_3OD$) $\delta$ 8.65 (s, 1H), 7.68 (s, 1H), 7.56 (d, *J* = 7.2 Hz, 1H), 7.03 (d, *J* = 7.2 Hz, 1H), 4.50-4.30 (m, 2H), 4.08-3.96 (m, 1H), 3.74-3.64 (m, 4H), 2.70 (t, *J* = 11.8 Hz, 2H), 2.23-2.16 (m, 2H), 1.46-1.35 (m, 2H), 1.31-1.24 (m, 12H); m/z $(ESI)^+$: 437.9 $[M+H]^+$. **Compound 421:** $^1$HNMR (400 MHz, $CD_3OD$) $\delta$ 8.55 (s, 1H), 7.74 (s, 1H), 7.59 (d, *J* = 7.2 Hz, 1H), 7.07 (d, *J* = 7.2 Hz, 1H), 4.63-4.36 (m, 2H), 4.32 (s, 1H), 4.01-3.93 (m, 2H), 3.73-3.64 (m, 2H), 2.70 (t, *J* = 11.8 Hz, 2H), 2.03-1.94 (m, 2H), 1.76-1.66 (m, 2H), 1.30-1.24 (m, 12H); m/z $(ESI)^+$: 437.9 $[M+H]^+$.

Referring to Example 1 and Example 3, the compounds in Table **3** were synthesized by substituting (2*R*,6*S*)-2,6-dimethylmorpholine with appropriate amine in Step 1 in Example 1, and/or substituting **3-3** with appropriate amine in Step 4 in Example 3. Compounds 527, 528, 561, and 562 without specified retention time RT were prepared using chiral starting materials.

**Table 3**

| Compound | Structure | m/z $(ESI)^+$ $[M+H]^+$ | RT(min)[a] |
|---|---|---|---|
| **424** | 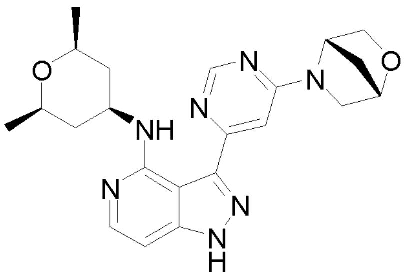 | **421.8** | 4.94[a] |
| **438** | 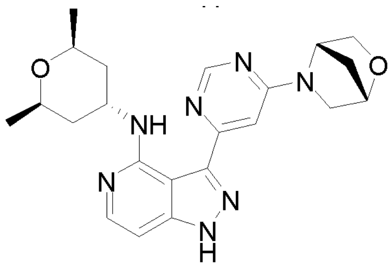 | **421.9** | 8.16[a] |

(continued)

| Compound | Structure | m/z (ESI)+ [M+H]+ | RT(min)[a] |
|---|---|---|---|
| **425** | 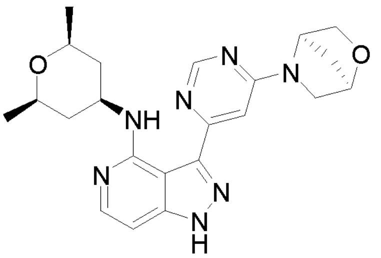 | **421.8** | 10.93[b] |
| **440** | 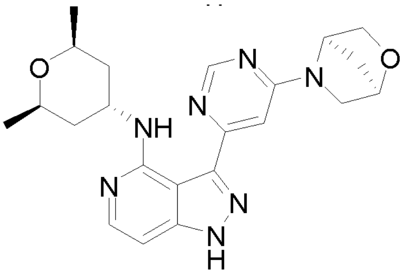 | **421.8** | 12.45[b] |
| **486** | 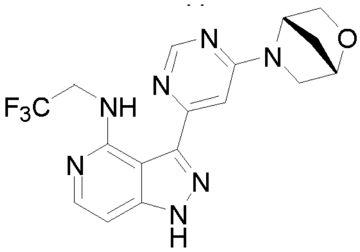 | **392.2** | |
| **487** | 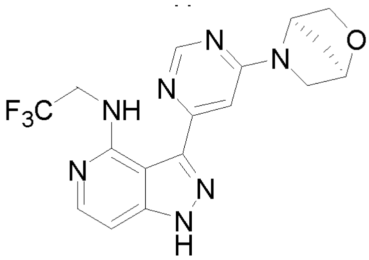 | **391.8** | |
| **519** | 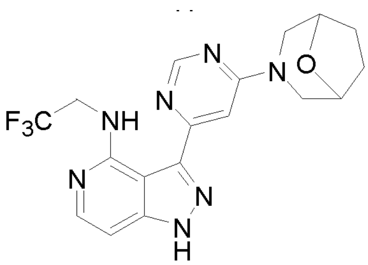 | **406.0** | |
| **520** | 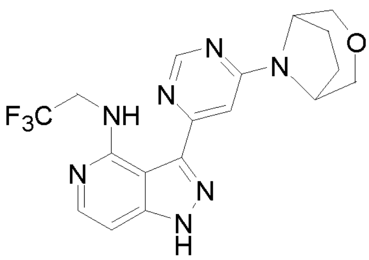 | **406.1** | |

(continued)

| Compound | Structure | m/z (ESI)$^+$ [M+H]$^+$ | RT(min)[a] |
|---|---|---|---|
| 527 | 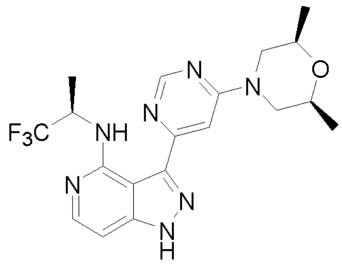 | 421.7 | |
| 528 | 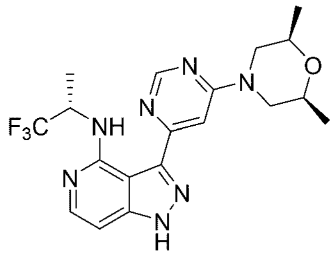 | 421.7 | |
| 561 | 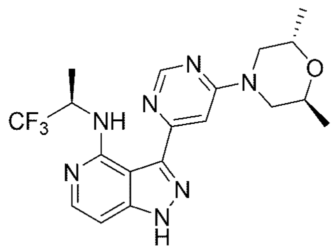 | 421.7 | |
| 562 | 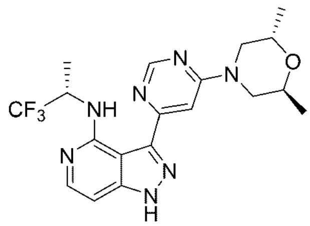 | 421.7 | |

[a]: SFC (chiral chromatography column chiralpak-IC column, 4.6 mm × 250 mm, 5 μm, eluent $CO_2$-EtOH (0.2% $NH_4OH$)).
[b]: Prep-HPLC (Waters, Gemini 5 μm, C18 150 × 21.2 mm, 20 mL/min, ACN/$H_2O$ (0.1% TFA) = 2%:98%, holding 6 min, then 23%:77%, holding 10 min).

**Example 4: Preparation of Compound 422 and Compound 445**

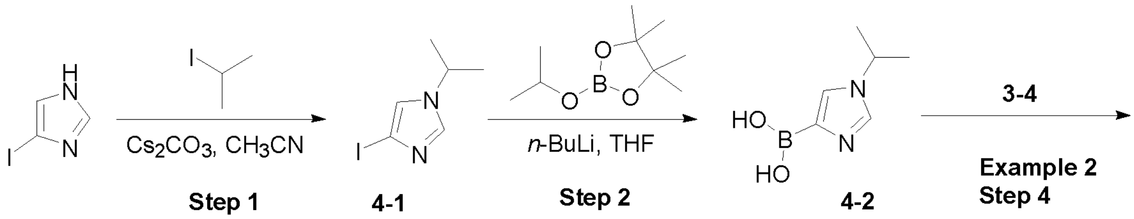


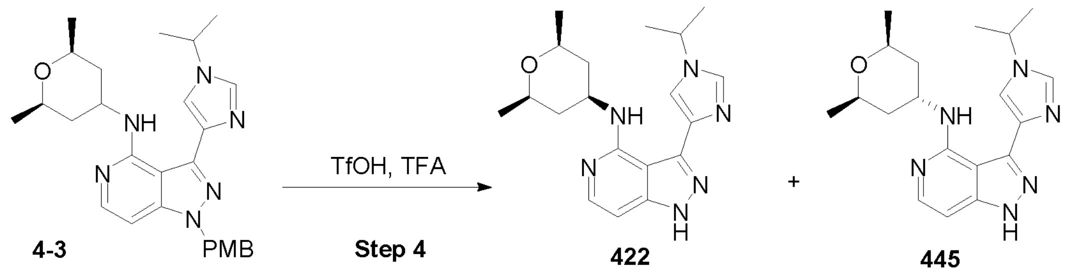


**Step 1: Synthesis of 4-iodo-1-isopropyl-1*H*-imidazole (4-1)**

$Cs_2CO_3$ (50 g, 0.15 mol) was added to a solution of 4-iodo-1*H*-imidazole (20 g, 0.1 mol) and 2-iodopropane (21 g, 0.12 mol) in $CH_3CN$ (50 mL). The resulting mixture was stirred at 60 °C overnight. The mixture was cooled to room temperature and filtered, and the filtrate was concentrated. The residue was dissolved in EtOAc, washed with brine, dried over anhydrous $Na_2SO_4$, filtered, and concentrated. The residue was purified by silica gel column chromatography (PE/EA = 1:1) to give compound 4-1 (20 g, 82%) in the form of a colorless oil. m/z $(ESI)^+$: 236.8 $[m+H]^+$.

**Step 2: Synthesis of (1-isopropyl-1*H*-imidazol-4-yl)boronic acid (4-2)**

A solution of n-BuLi in n-hexane (2.4 M, 16 mL, 38 mmol) was added slowly and dropwise to a solution of compound 4-1 (3 g, 13 mmol) and 2-isopropoxy-4,4,5,5-tetramethyl-1,3,2-dioxabenzaldehyde (7.1 g, 38 mmol) in THF (20 mL) at -75 °C under nitrogen atmosphere. After the dropwise addition was completed, the mixture was stirred at -75 °C for 2 h. The mixture was added with $NH_4Cl$ (saturated aqueous solution, 50 mL) to quench the reaction. The mixture was extracted with EtOAc, and the organic layer was washed with brine, dried over anhydrous $Na_2SO_4$, filtered, and concentrated to give a residue. The residue was purified by silica gel column chromatography (DCM/MeOH = 10:1) to give compound 4-2 (1.7 g, 87%) in the form of a white solid. m/z $(ESI)^+$: 155.2 $[m+H]^+$.

**Step 3: Synthesis of *N*-((2*R*,6*S*)-2,6-dimethyltetrahydro-2*H*-pyran-4-yl)-3-(1-isopropyl-1*H*-imidazol-4-yl)-1-(4-methoxybenzyl)-1*H*-pyrazolo[4,3-*c*]pyridine-4-amine (4-3)**

Similar to the method of Step 4 in Example 2, compounds 4-2 and 3-4 were used to synthesize *N*-((2*R*,6*S*)-2,6-dimethyltetrahydro-2H-pyran-4-yl)-3-(1-isopropyl-1H-imidazol-4-yl)-1-(4-methoxy benzyl)-1*H*-pyrazolo[4,3-*c*]pyridine-4-amine (4-3) in the form of a white solid. m/z $(ESI)^+$: 475.2 $[m+H]^+$.

**Step 4: Synthesis of N-((2*R*,4*R*,6*S*)-2,6-dimethyltetrahydro-2*H*-pyran-4-yl)-3-(1-isopropyl-1*H*-imidazol-4-yl)-1*H*-pyrazolo[4,3-*c*]pyridine-4-amine (422) and N-((2*R*,4*S*,6*S*)-2,6-dimethyl tetrahydro-2*H*-pyran-4-yl)-3-(1-isopropyl-1*H*-imidazol-4-yl)-1*H*-pyrazolo[4,3-*c*]pyridine-4-am ine (445)**

TfOH (0.5 mL) was added to a solution of intermediate 4-3 (200 mg, 0.42 mol) in TFA (10 mL). The mixture was stirred at room temperature for 1 h. The mixture was concentrated and diluted in EtOAc, washed with 1 M aqueous NaOH solution and brine, dried over anhydrous $Na_2SO_4$, filtered, and concentrated to give a residue. The residue was purified by silica gel column chromatography (PE/EA = 1:9), and then separated by SFC (chiral chromatography column chiralpak IC column, 4.6 mm × 250 mm, 5 μm, eluting with $CO_2$-EtOH (0.2% NHaOH)) to give compound 422 (9 mg) at RT = 15.6 min and compound 445 (3 mg) at RT = 10.09 min in the form of white solids.

**Compound 422:** $^1$HNMR (400 MHz, $CD_3OD$) $\delta$ 7.95 (s, 1H), 7.79 (s, 1H), 7.59 (d, $J$ = 6.6 Hz, 1H), 6.76 (d, $J$ = 6.6 Hz, 1H), 4.60-4.52 (m, 1H), 4.17-4.07 (m, 1H), 3.75-3.65 (m, 2H), 2.23-2.13 (m, 2H), 1.58 (d, $J$ = 6.6 Hz, 6H), 1.38-1.31 (m, 2H), 1.27 (d, $J$ = 6.1 Hz, 1H); m/z $(ESI)^+$: 355.2 $[M+H]^+$.

**Compound 445:** $^1$HNMR (400 MHz, $CD_3OD$) 7.88 (s, 1H), 7.84 (s, 1H), 7.61 (d, $J$ = 6.6 Hz, 1H), 6.78 (d, $J$ = 6.6 Hz, 1H), 4.65-4.55 (m, 1H), 4.45-4.37 (m, 1H), 4.15-4.05 (m, 2H), 1.98-1.88 (m, 2H), 1.73-1.63 (m, 2H), 1.59 (d, $J$ = 6.6 Hz,

6H), 1.25 (d, *J* = 6.1 Hz, 1H); m/z (ESI)$^+$: 355.2 [M+H]$^+$.

Referring to Examples 3 and 4, the compounds shown in Table 4 can be prepared by substituting amine **3-3** with appropriate amine in Step 4 in Example 3.

**Table 4**

| Compound | Structure | m/z (ESI)$^+$ M+H$^+$ |
|---|---|---|
| **426** | 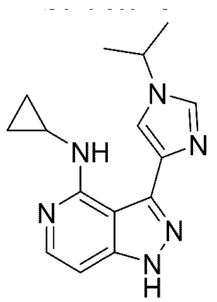 | 283.1 |
| **516** | 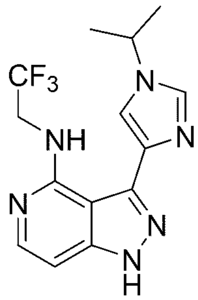 | 324.8 |

**Example 5: Preparation of Compounds 423 and 439**

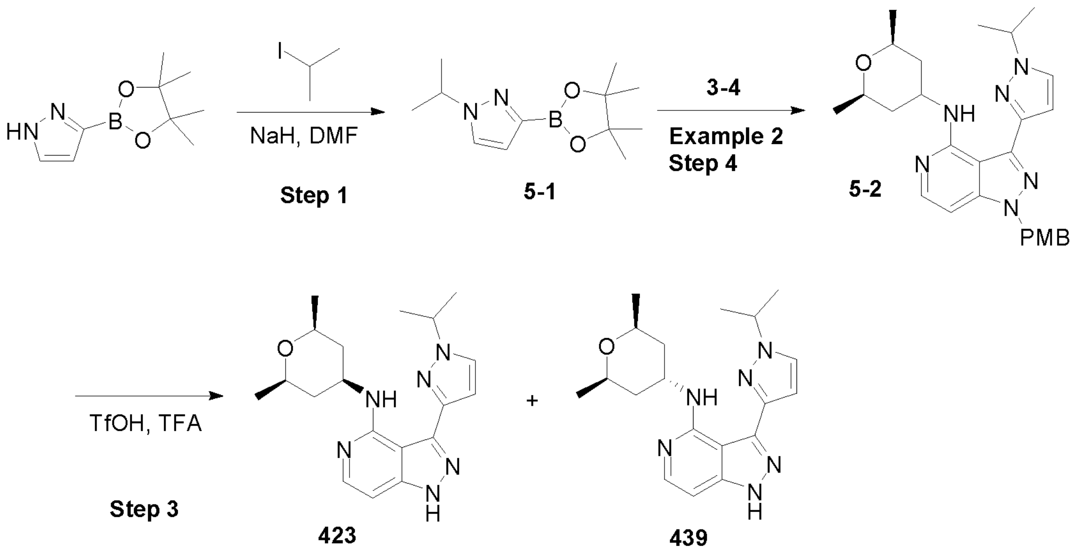

5-1
5-2
423
439

**Step 1: Synthesis of 1-isopropyl-3-(4,4,5,5-tetramethyl-1,3,2-dioxabenzaldehyde-2-yl)-1*H*-pyrazole (5-1)**

3-(4,4,5,5-Tetramethyl-1,3,2-dioxabenzofuran-2-yl)-1*H*-pyrazole (1.04 g, 5.36 mmol) was added to a suspension of NaH (60%, 0.24 g, 6 mmol) in DMF (10 mL) at 0 °C under nitrogen atmosphere. The mixture was stirred at 30 °C for 30 min. The mixture was then cooled to 0 °C, and 2-iodopropane (0.8 mL, 8 mmol) was added dropwise. The resulting mixture was stirred at room temperature for additional 30 min. The mixture was added with a saturated aqueous $NH_4Cl$ solution to quench the reaction, and then extracted with ethyl acetate. The organic layer was washed with brine, dried over

anhydrous $Na_2SO_4$, filtered, and concentrated to give a residue. The residue was purified by silica gel column chromatography (PE/EA = 4:1) to give compound 5-1 (550 mg, 43%) in the form of a colorless oil. $^1$HNMR (300 MHz, $CD_3OD$) $\delta$ 7.67 (d, *J* = 2.3 Hz, 1H), 6.59 (d, *J* = 2.3 Hz, 1H), 4.66-4.52 (m, 1H), 1.48 (d, *J* = 6.7 Hz, 6H), 1.19(s, 12H).

**Step 2: Synthesis of N-((2*R*,6*S*)-2,6-dimethyltetrahydro-2*H*-pyran-4-yl)-3-(1-isopropyl-1*H*-pyrazol-3-yl)-1-(4-methoxybenzyl)-1*H*-pyrazolo[4,3-*c*]pyridine-4-amine (5-2)**

Similar to the method of Step 4 in Example 2, compounds 5-1 and 3-4 were used to synthesize *N*-((2*R*,6*S*)-2,6-dimethyltetrahydro-2*H*-pyran-4-yl)-3-(1-isopropyl-1*H*-imidazol-4-yl)-1-(4-methoxy benzyl)-1*H*-pyrazolo[4,3-*c*]pyridine-4-amine (5-2) in the form of a white solid. m/z (ESI)$^+$: 475.2 [M+H]$^+$.

**Step 3: Synthesis of *N*-((2*R*,4*R*,6*S*)-2,6-dimethyltetrahydro-2*H*-pyran-4-yl)-3-(1-isopropyl-1*H*-pyrazol-3-yl)-1*H*-pyrazolo[4,3-*c*]pyridine-4-amine (423) and N-((2*R*,4*S*,6*S*)-2,6-dimethyl tetrahydro-2*H*-pyran-4-yl)-3-(1-isopropyl-1*H*-pyrazol-3-yl)-1*H*-pyrazolo[4,3-*c*]pyridine-4-ami ne (439)**

TfOH (0.5 mL) was added to a solution of intermediate 5-2 (200 mg, 0.42 mmol) in TFA (10 mL). The mixture was stirred at room temperature for 1 h. The mixture was concentrated and diluted in EtOAc, washed with 1 M aqueous NaOH solution and brine, dried over anhydrous $Na_2SO_4$, filtered, and concentrated to give a residue. The residue was purified by silica gel column chromatography (DCM/MeOH = 10:1), and then separated by SFC (chiral chromatography column chiralpak IC column, 4.6 mm $\times$ 250 mm, 5 $\mu$m, eluting with $CO_2$-IPA (0.2% $NH_4OH$)) to give compound 423 (50 mg) at RT = 10.55 min and compound 439 (17 mg) at RT = 7.90 min in the form of white solids.

**Compound 423:** $^1$HNMR (400 MHz, $CD_3OD$) $\delta$ 7.86 (d, *J* = 2.4 Hz, 1H), 7.61 (d, *J* = 6.9 Hz, 1H), 6.95-6.90 (m, 2H), 4.76-4.66 (m, 1H), 4.21-4.13 (m, 1H), 3.77-3.68 (m, 2H), 2.28-2.21 (m, 2H), 1.64 (d, *J* = 6.7 Hz, 6H), 1.44-1.33 (m, 2H), 1.29 (d, *J* = 6.2 Hz, 6H); m/z (ESI)$^+$: 354.9 [M+H]$^+$.

**Compound 439:** $^1$HNMR (400 MHz, $CD_3OD$) $\delta$ 7.87 (d, *J* = 2.5 Hz, 1H), 7.70 (d, *J* = 6.4 Hz, 1H), 6.96 (d, *J* = 2.5 Hz, 1H), 6.76 (d, *J* = 6.4 Hz, 1H), 4.67-4.57 (m, 1H), 4.54-4.49 (m, 1H), 4.06-3.97 (m, 2H), 2.04-1.97 (m, 2H), 1.67-1.62 (m, 2H), 1.60 (d, *J* = 6.8 Hz, 6H), 1.23 (d, *J* = 6.2 Hz, 6H); m/z (ESI)$^+$: 354.9 [M+H]$^+$.

Referring to Examples 3 and 5, the compounds shown in Table 5 can be prepared by substituting amine 3-3 with appropriate amine in Step 4 in Example 3.

**Table 5**

| Compound | Structure | m/z (ESI)$^+$ [M+HI$^+$ |
|---|---|---|
| **427** | 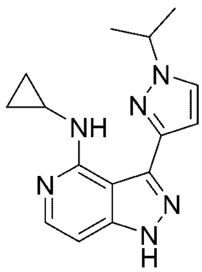 | 282.9 |
| **515** | 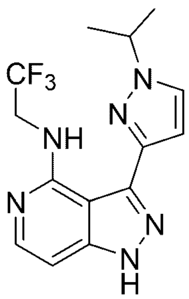 | 324.9 |

**Example 6: Preparation of Compound 523**

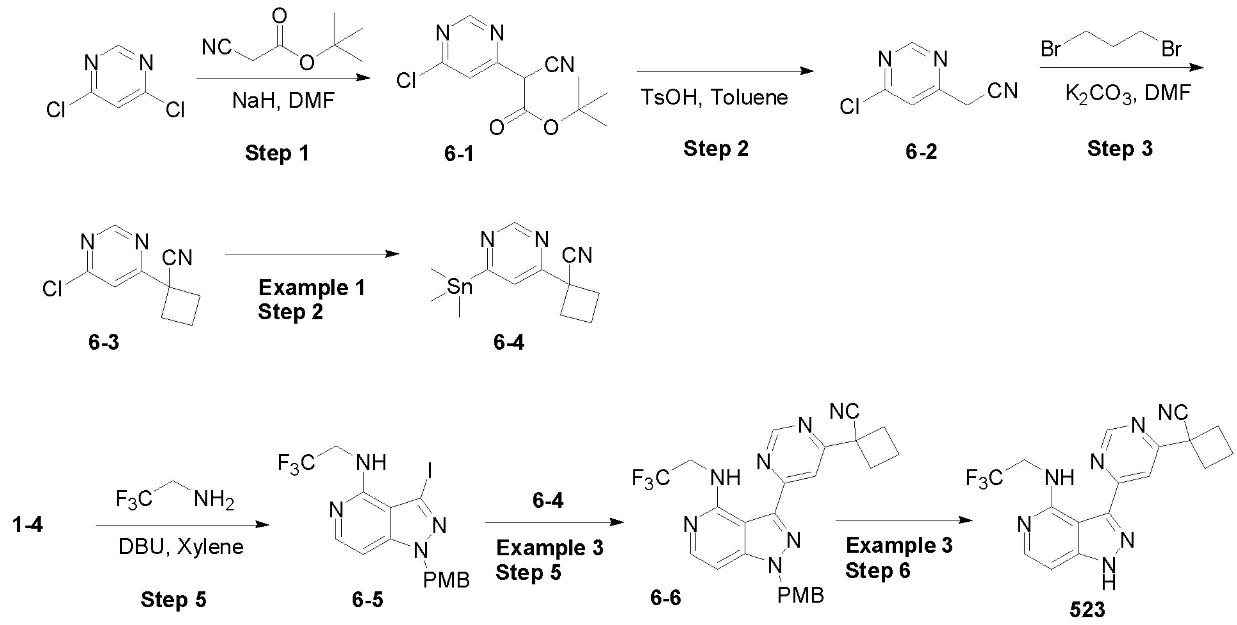


**Step 1: Synthesis of *tert*-butyl 2-(6-chloropyrimidin-4-yl)-2-cyanoacetate (6-1)**

Sodium hydride (60%, 6.71 g, 0.168 mol) was added to a solution of 4,6-dichloropyrimidine (10 g, 0.067 mol) and *tert*-butyl 2-cyanoacetate (23.7 g, 0.168 mol) in THF (100 mL) at 0 °C under nitrogen atmosphere. The reaction mixture was stirred at room temperature for 2 h. The mixture was added with a saturated aqueous $NH_4Cl$ solution to quench the reaction, and then adjusted to pH 2-3 with 1 M HCl aqueous solution. The mixture was extracted with EtOAc twice, and the combined organic layers were washed with brine, dried over anhydrous $Na_2SO_4$, filtered, and concentrated to give compound 6-1 (16 g, 94%) in the form of a white solid. m/z(ESI)+: 253.9$[m+H]^+$.

**Step 2: Synthesis of 2-(6-chloropyrimidin-4-yl)acetonitrile (6-2)**

*p*-Toluenesulfonic acid monohydrate (0.82 g, 0.0043 mol) was added to a solution of intermediate 6-1 (11 g, 0.043 mol) in toluene (100 mL). The mixture was stirred at 100 °C for 3 h. The mixture was cooled to room temperature and diluted with EtOAc. The mixture was washed with a saturated aqueous $NaHCO_3$ solution and brine, dried over anhydrous $Na_2SO_4$, filtered, and concentrated to give a residue. The residue was purified by silica gel column chromatography (PE/EA = 7:3) to give compound 6-2 (5.5 g, 84%) in the form of a yellow solid. m/z(ESI)+: 154.0$[m+H]^+$.

**Step 3: Synthesis of 1-(6-chloropyrimidin-4-yl)cyclobutane-1-carbonitrile (6-3)**

A solution of intermediate 6-2 (612 mg, 4 mmol) and 1,3-dibromopropane (965 mg, 4.8 mmol) in DMF (10 mL) was added dropwise to a suspension of potassium carbonate (1.38 g, 10 mmol) in DMF (10 mL) in an ice-water bath. The reaction mixture was stirred at room temperature for 3 h. The mixture was diluted with EtOAc, then washed with brine, dried over anhydrous $Na_2SO_4$, filtered, and concentrated to give a residue. The residue was purified by silica gel column chromatography (PE/EA = 1:1) to give compound 6-3 (600 mg, 78%) in the form of a yellow solid. m/z(ESI)+: 194.0 $[m+H]^+$.

**Step 4: Synthesis of 1-(6-(trimethylstannyl)pyrimidin-4-yl)cyclobutane-1-carbonitrile (6-4)**

Similar to the method of Step 2 in Example 1, compound 6-3 was used to synthesize 1-(6-(trimethylstannyl) pyrimidin-4-yl)cyclobutane-1-carbonitrile (6-4) in the form of a yellow solid. m/z(ESI)+: 323.9 $[m+H]^+$.

**Step 5: Synthesis of 3-iodo-1-(4-methoxybenzyl)-*N*-(2,2,2-trifluoroethyl)-1*H*-pyrazolo[4,3-c] pyridine-4-amine (6-5)**

A suspension of intermediate 1-4 (30 g, 0.075 mol), 2,2,2-trifluoroethylamine hydrochloride (30.5 g, 0.225 mol) and DBU (23 g, 0.15 mmol) in xylene (250 mL) was sealed and heated to 190 °C in an oil bath and stirred for 3 h. The mixture was cooled to room temperature and diluted in EtOAc. The mixture was washed with brine, dried over anhydrous $Na_2SO_4$, filtered, and concentrated to give a residue. The residue was stirred in MTBE for 30 min at room temperature and filtered to

give compound 6-5 (24 g, 69%) in the form of a white solid. m/z(ESI)+: 463.1 [m+H]$^+$.

**Step 6: Synthesis of 1-(6-(1-(4-methoxybenzyl)-4-(2,2,2-trifluoroethyl)amino)-1*H*-pyrazolo [4,3-c]pyridin-3-yl) pyrimidin-4-yl)cyclobutane-1-carbonitrile (6-6)**

Similar to the method of Step 5 in Example 3, compounds 6-4 and 6-5 were used to synthesize 1-(6-(1-(4-methoxybenzyl)-4-(2,2,2-trifluoroethyl)amino)-1*H*-pyrazolo[4,3-c]pyridin-3-yl)pyrimidi n-4-yl)cyclobutane-1-carbonitrile (6-6) in the form of a yellow solid. m/z(ESI)+:493.9[m+H]+.

**Step 7: Synthesis of 1-(6-(4-((2,2,2-trifluoroethyl)amino)-1*H*-pyrazolo[4,3-c]pyridin-3-yl) pyrimidin-4-yl)cyclobutane-1-carbonitrile (523)**

Similar to the method of Step 6 in Example 3, compound 6-6 was used to synthesize 1-(6-(4-((2,2,2-trifluoroethyl) amino)-1*H*-pyrazolo[4,3-c]pyridin-3-yl)pyrimidin-4-yl)cyclobutane-1-carbonitrile (523) in the form of a yellow solid. $^1$HNMR (400 MHz, DMSO-d6) $\delta$ 13.65 (s, 1H), 10.26 (s, 1H), 8.08 (s, 1H), 7.80 (d, *J* = 6.0 Hz, 1H), 7.32 (s, 1H), 6.80 (d, *J* = 6.0 Hz, 1H), 4.48-4.35 (m, 2H), 3.92-3.81 (m, 2H), 2.34-2.27 (m, 2H), 1.90-1.80 (m, 2H); m/z (ESI)$^+$: 373.8 [M+H]$^+$.

Referring to Example 6, the compounds shown in Table 6 were prepared by substituting 1,3-dibromopropane with appropriate halohydrocarbon in Step 2, or by substituting 2,2,2-trifluoroethylamine with appropriate amine in Step 5.

**Table 6**

| Compound | Structure | m/z (ESI)$^+$ [M+HI$^+$ |
|---|---|---|
| **524** | 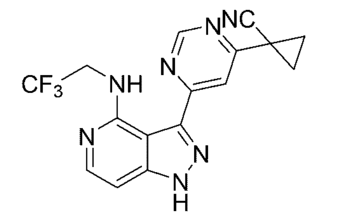 | 360.0 |
| **525** | 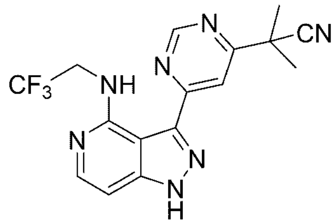 | 361.8 |
| **508** | 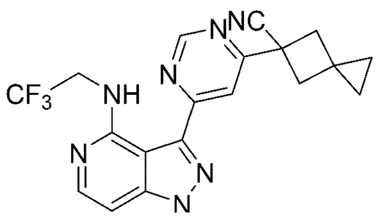 | 399.8 |
| **529** | 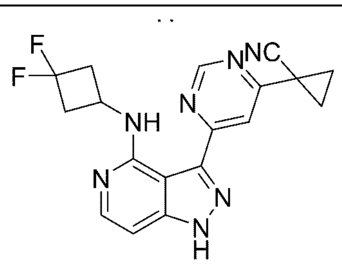 | 367.8 |

**Example 7: Preparation of Compounds 521 and 509**

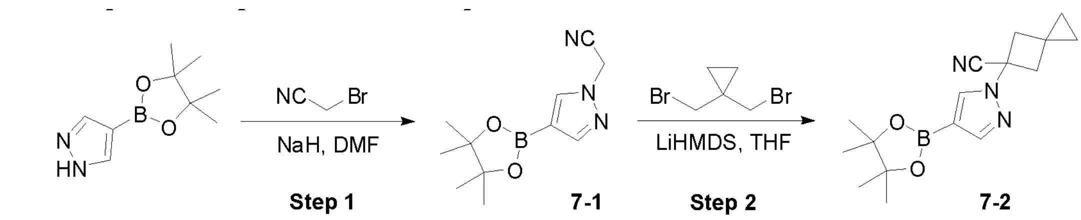


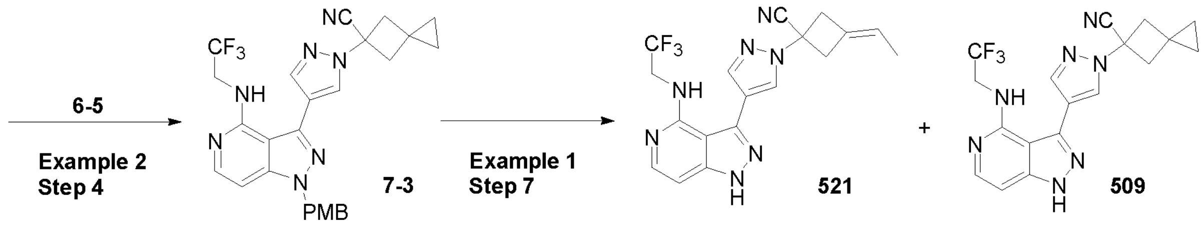


**Step 1: Synthesis of 2-(4,4,5,5-tetramethyl-1,3,2-dioxabenzaldehyde-2-yl)-1*H*-pyrazol-1-yl) acetonitrile (7-1)**

Sodium hydride (60%, 4.12 g, 0.103 mol) was added to a solution of 4-(4,4,5,5-tetramethyl-1,3,2-dioxabenzofuran-2-yl)-1*H*-pyrazole (10 g, 0.052 mol) in DMF (150 mL) under stirring at 0 °C. After the mixture was stirred for 30 min, 2-bromoacetonitrile (12.35 g, 0.103 mol) was added. The mixture was stirred at room temperature overnight. The mixture was added with a saturated aqueous $NH_4Cl$ solution to quench the reaction, and extracted with ethyl acetate twice. The combined organic layers were washed with brine, dried over anhydrous $Na_2SO_4$, filtered, and concentrated to give a residue. The residue was purified by silica gel column chromatography (DCM/MeOH = 97:3) to give compound 7-1 (2.0 g, 17%) in the form of an off-white solid. m/z $(ESI)^+$: 234.0$[m+H]^+$.

**Step 2: Synthesis of 5-(4-(4,4,5,5-tetramethyl-1,3,2-dioxabenzaldehyde-2-yl)-1*H*-pyrazol-1-yl) spiro[2.3]hexane-5-carbonitrile (7-2)**

Under nitrogen atmosphere, a solution of intermediate 7-1 (2.05 g, 8.8 mmol) and 1,1-bis(bromomethyl) cyclopropane (2.0 g, 8.8 mmol) in THF (50 mL) was added dropwise to a solution of LiHMDS (1 M, 43 mL, 43 mmol) in THF (150 mL) at -5 °C to 0 °C. The mixture was stirred at this temperature for 10 min, and then the reaction was quenched with formic acid. The mixture was concentrated to give a residue. The residue was purified by silica gel column chromatography (DCM/MeOH = 99:1) to give compound 7-2 (170 mg, 26%) in the form of a colorless oil. m/z $(ESI)^+$: 300.0 $[m+H]^+$.

**Step 3: Synthesis of 5-(4-(1-(4-methoxybenzyl)-4-(2,2,2-trifluoroethyl)amino)-1*H*-pyrazolo [4,3-*c*]pyridin-3-yl)-1*H*-pyrazol-1-yl)spiro[2.3]hexane-5-carbonitrile (7-3)**

Similar to the method of Step 4 in Example 2, compounds 7-2 and 6-5 were used to synthesize 5-(4-(1-(4-methoxybenzyl)-4-(2,2,2-trifluoroethyl)amino)-1*H*-pyrazolo[4,3-*c*]pyridin-3-yl)-1*H*-pyra zol-1-yl)spiro[2.3]hexane-5-carbonitrile (7-3) in the form of a yellow solid. $^1$HNMR (300 MHz, $CDCl_3$) $\delta$ 8.04 (s, 1H), 7.96 (s, 1H), 7.87 (d, *J* = 6.1 Hz, 1H), 7.20-7.15 (m, 2H), 6.86-6.79 (m, 2H), 6.68 (d, *J* = 6.1 Hz, 1H), 5.43 (s, 2H), 5.16 (t, *J* = 6.3 Hz, 1H), 4.37-4.24 (m, 2H), 3.76 (s, 3H), 3.34-3.26 (m, 2H), 3.03-2.95 (m, 2H), 0.85-0.77 (m, 2H), 0.67-0.59 (m, 2H); m/z $(ESI)^+$: 507.7 $[M+H]^+$.

**Step 4: Synthesis of 3-ethylidene-1-(4-(4-((2,2,2-trifluoroethyl)amino)-1*H*-pyrazolo[4,3-*c*] pyridin-3-yl)-1*H*-pyrazol-1-yl)cyclobutane-1-carbonitrile (521) and 5-(4-(4-((2,2,2,2-trifluoroethyl)amino)-1*H*-pyrazolo[4,3-*c*]pyridin-3-yl)-1*H*-pyrazol-1-yl)spiro [2.3]hexane-5-carbonitrile (509)**

Similar to the method of Step 7 in Example 1, compound 7-3 was used to synthesize 3-ethylidene-1-(4-(4-((2,2,2-trifluoroethyl)amino)-1*H*-pyrazolo[4,3-*c*]pyridin-3-yl)-1*H*-pyrazol-1-yl )cyclobutane-1-carbonitrile (521) and 5-(4-(4-((2,2,2-trifluoroethyl)amino)-1*H*-pyrazolo[4,3-c]pyridin-3-yl)-1*H*-pyrazol-1-yl)spiro[2.3]he xane-5-carbonitrile (509) in the form of white solids.

**Compound 521:** RT = 8.3 min (preparative HPLC (GILSON, Gemini 5 $\mu$m, C18 150 $\times$ 21.2 mm, 20 mL/min, ACN/$H_2O$ (0.05% $NH_4OH$) = 35%:65%-60%:40%, within 10 min)); $^1$HNMR (400 MHz, $CD_3OD$) $\delta$ 8.26 (s, 1H), 7.99 (s, 1H), 7.84 (d, *J* = 6.2 Hz, 1H), 6.89 (d, *J* = 6.2 Hz, 1H), 5.60-5.52 (m, 1H), 4.28 (q, *J* = 9.5 Hz, 2H), 3.79-3.64 (m, 4H), 1.66-1.60 (m, 3H); m/z $(ESI)^+$: 387.8 $[M+H]^+$.

**Compound 509:** RT = 7.7 min (preparative HPLC (GILSON, Gemini 5 μm, C18 150 × 21.2 mm, 20 mL/min, ACN/$H_2O$ (0.05% $NH_4OH$) = 35%:65%-60%:40%, within 10 min)); $^1$HNMR (400 MHz, $CD_3OD$) δ 8.30 (s, 1H), 7.99 (s, 1H), 7.84 (d, *J* = 6.2 Hz, 1H), 6.89 (d, *J* = 6.2 Hz, 1H), 5.60-5.52 (m, 1H), 4.29 (q, *J* = 9.5 Hz, 2H), 3.30-3.26 (m, 2H), 3.04-2.98 (m, 2H), 0.83-0.77 (m, 2H), 0.66-0.59 (m, 2H); m/z (ESI)$^+$: 387.9 [M+H]$^+$.

**Example 8: Preparation of Compound 526**

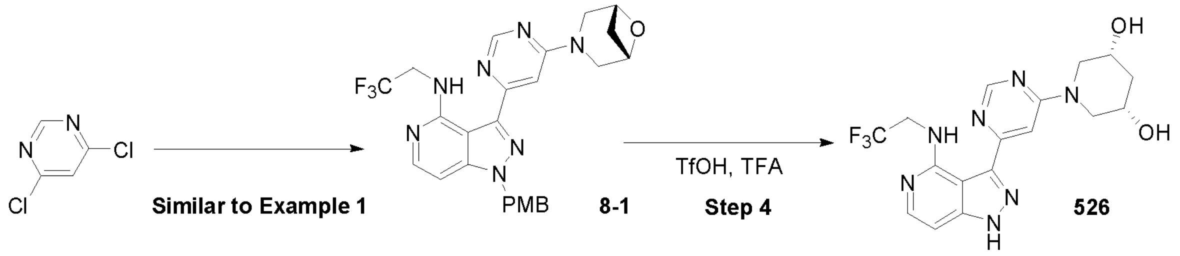


**Step 1: Synthesis of 3-(6-((1*R*,5*S*)-6-oxa-3-azabicyclo[3.1.1]heptan-3-yl)pyrimidin-4-yl)-1-(4-methoxybenzyl)-*N*-(2,2,2-trifluoroethyl)-1*H*-pyrazolo[4,3-c]pyridine-4-amine (8-1)**

Similar to the method in Example 1, 4,6-dichloropyrimidine was used to synthesize 3-(6-((1*R*,5*S*)-6-oxa-3-azabicyclo[3.1.1]heptan-3-yl)pyrimidin-4-yl)-1-(4-methoxybenzyl)-*N*-(2,2,2-trifluoroethyl)-1*H*-pyrazolo[4,3-*c*]pyridine-4-amine (8-1) by using appropriate reagents in appropriate steps.

**Step 2: Synthesis of (3*S*,5*R*)-1-(6-(4-((2,2,2-trifluoroethyl)amino)-1*H*-pyrazolo[4,3-c]pyridin-3-yl)pyrimidin-4-yl) piperidine-3,5-diol (526)**

TfOH (0.5 mL) was added to a solution of compound 8-1 (90 mg, 0.176 mmol) in TFA (10 mL). The resulting mixture was stirred at room temperature for 1 h, then concentrated, and added with DCM for dilution. The mixture was then washed with 1 M aqueous NaOH solution and brine. The separated organic layer was dried over anhydrous $Na_2SO_4$, filtered, and concentrated to give a residue. The residue was purified by preparative TLC (DCM/MeOH = 5:1) and by preparative HPLC (Gilson, Gemini 5 μm, C18 150 × 21.2 mm, 20 mL/min, ACN/$H_2O$ (0.1% FA) = 35%:65%-50%:50% in 10 min) to give compound **526** (20 mg) in the form of a white solid. $^1$HNMR (400 MHz, $CD_3OD$) δ 8.58 (d, *J* = 1.0 Hz, 1H), 7.77 (d, *J* = 6.3 Hz, 1H), 7.71 (d, *J* = 1.0 Hz, 1H), 6.87 (d, *J* = 6.3 Hz, 1H), 4.39 (q, *J* = 9.5 Hz, 2H), 4.16-4.08 (m, 2H), 4.05-3.90 (m, 2H), 3.68-3.58 (m, 2H), 1.95 (t, *J* = 5.4 Hz, 2H). m/z (ESI)$^+$: 410.0 [M+H]$^+$.

**Example 9: Preparation of Compound 522**

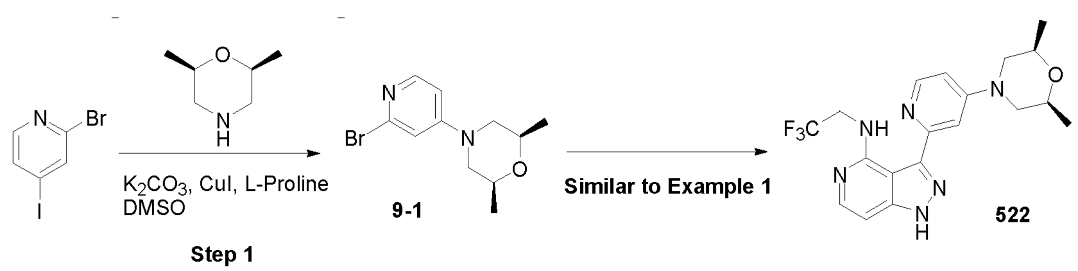


**Step 1: Synthesis of (2*R*,6*S*)-4-(2-bromopyridin-4-yl)-2,6-dimethylmorpholine (9-1)**

Copper(I) iodide (70 mg, 0.352 mmol) and L-proline (80 mg, 0.704 mmol) were added to a suspension of 2-bromo-4-iodopyridine (1.0 g, 3.52 mmol), (2*R*,6*S*)-2,6-dimethylmorpholine (400 mg, 3.52 mmol), and $K_2CO_3$ (1.5 g, 10.5 mmol) in DMSO (20 mL) under nitrogen atmosphere. The resulting mixture was degassed and purged with nitrogen thrice, and then stirred at 60 °C for 1 h. The reaction mixture was diluted in EtOAc and washed with brine. The separated organic layer was dried over anhydrous $Na_2SO_4$, filtered, and concentrated to give a residue. The residue was purified by silica gel

column chromatography (DCM/MeOH = 99:1) to give compound 9-1 (400 mg, 42%) in the form of a white solid. m/z (ESI)+: 270.8, 272.8 [m+H]+.

**Step 2: Synthesis of 3-(4-((2S,6R)-2,6-dimethylmorpholinyl)pyridin-2-yl)-N (2,2,2-trifluoro ethyl)-1*H*-pyrazolo [4,3-c]pyridine-4-amine (522)**

Similar to the method in Example 1, compound 9-1 was used to synthesize 3-(4-((2*S*,6*R*)-2,6-dimethylmorpholinyl)pyridin-2-yl)-*N*-(2,2,2-trifluoroethyl)-1*H*-pyrazolo[4,3-c]py ridine-4-amine (522) by using appropriate reagents in appropriate steps. $^1$HNMR (400 MHz, CDC1$_3$) $\delta$ 8.25 (d, *J* = 6.1 Hz, 1H), 7.87 (d, *J* = 6.1 Hz, 1H), 7.76 (d, *J* = 2.6 Hz, 1H), 6.71 (dd, *J* = 6.1, 2.6 Hz, 2H), 4.50-4.36 (m, 2H), 3.81-3.71 (m, 4H), 2.66-2.58 (m, 2H), 1.30 (d, *J* = 6.2 Hz, 6H. m/z (ESI)+: 406.8 [M+H]+.

**Preparation Example: Synthesis of Control Compound**

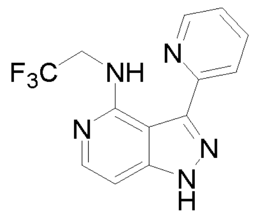

Control compound

The control compound described above was synthesized according to the method in Example 1, except that: Step 1 was not performed, Step 2 was performed using 2-chloropyridine; compound 1-2 in Example 1 was replaced by 2-(trimethylstannyl)pyridine prepared; and cyclopropylamine was replaced by $CF_3CH_2NH_2$ in Step 5 to give the control compound.

**Example 10: Kinase Inhibition Assay**

**Materials**

Kinase LRRK2 G2019S protein was derived from Carna, LRRKtide was derived from Signalchem, ATP was derived from Promega, and DMSO was derived from Sigma.

**Kinase assay method**

All assays were performed at room temperature. The test compounds were dissolved and diluted, and then added to a 384-well plate. 2.5 μL of 2× kinase reaction buffer was added, and the mixture was centrifuged. 2.5 μL of a mixture of 2× substrate and ATP was added, and the mixture was centrifuged. 4 μL of ADP-Glo reagent was added, and the mixture was incubated at room temperature for 40 min. 8 μL of LRRKtide was added, and the mixture was incubated at room temperature for 40 min. Then, the data were read using the Envision 2104 multi-label Reader.

**Results**

The $IC_{50}$ values for LRRK2 G2019S inhibition by compounds of the examples of the present disclosure are shown in Table 3 below.

Table 3

| Compound | LRRK2 $IC_{50}$ (G2019S, nM) |
| --- | --- |
| Control compound | 12.0 |
| 447 | 2.8 |
| 527 | 1.2 |

(continued)

| Compound | LRRK2 $IC_{50}$ (G2019S, nM) |
|---|---|
| 391 | 9.5 |
| 407 | 2.2 |
| 424 | 3.7 |
| 425 | 1.9 |
| 440 | 36.5 |
| 486 | 3.1 |
| 487 | 2.4 |
| 515 | 27.7 |
| 520 | 4.8 |
| 523 | 2.4 |
| 526 | 5.1 |
| 528 | 1.6 |
| 561 | 1.3 |
| 379 | 3.2 |
| 473 | 2.8 |

**Example 11: HEK293 Cell Kinase Inhibition Assay**

HEK293 cells were cultured, and collected by adding trypsin. Culture medium was added, and the cell density was adjusted to $2 \times 10^5$/mL. Liposome-DNA complexes were constructed and mixed with HEK293 cells at a ratio of 1:20. The mixture was added to a 384-well plate, and incubated at 37 °C for 20-30 h. A mixed solution of enzyme substrate and the test compound was added, and the mixture was incubated at room temperature for 2-3 min. NanoBRET reagent (Promega) was added, and bioluminescence was measured within 10 min using the microplate reader (Envision 2104) at 450 nm for the donor luciferase protein (donor) and 610 nm for the acceptor fluorescent group (acceptor). The BRET ratio for the test compounds was calculated using the following formula, including a background correction option.

$$\text{BRET Ratio} = \left[\left(\frac{\text{Acceptor}_{\text{sample}}}{\text{Donor}_{\text{sample}}}\right) - \left(\frac{\text{Acceptor}_{\text{no-tracer control}}}{\text{Donor}_{\text{no-tracer control}}}\right)\right] \times 1{,}000$$

$\text{Acceptor}_{\text{sample}}$: 610 nm fluorescence with the test compound;
$\text{Donor}_{\text{Sample:}}$ 450 nm fluorescence with the test compound;
$\text{Acceptor}_{\text{no-tracer control}}$ : 610 nm fluorescence of background (without the test compound);
$\text{Donor}_{\text{no-tracer control}}$: 450 nm fluorescence of background (without the test compound)

The inhibitory activity of the test compounds on cellular LRRK2 enzyme was calculated by the following formula:

Percentage inhibition rate = 100 - (BRET ratio of the test compound - mean BRET ratio of all microplate positive controls)/(mean BRET ratio of all microplate negative controls - mean BRET ratio of all microplate positive controls) $\times$ 100.

The $IC_{50}$ values of the test compounds against cellular LRRK2 enzyme were calculated using Graphpad software. Non-linear regression fitting (dose-effect - variable slope method) was performed on the percentage inhibition rate versus logarithmic concentration curve of the compounds.

$$Y=Bottom + (Top-Bottom)/(1+10^{\wedge}((LogIC50-X)*Hill\ Slope))$$

X: logarithmic inhibitor concentration; Y: percentage inhibition rate; Bottom: a minimum value; Top: a maximum value; Hill Slope: hill slope

**Results**

The $IC_{50}$ values for the inhibition of LRRK2, LRRK2-2019S, and LRRK2-R1441C in HEK293 cells by the compounds in some examples of the present disclosure are summarized in the following table:

| Compound | LRRK2 $IC_{50}$ (nM) | LRRK2 $IC_{50}$ (G2019S, nM) | LRRK2 $IC_{50}$ (R1441C, nM) |
|---|---|---|---|
| 379 | 137.1 | 64.4 | 188.2 |
| 447 | 246.5 | 97.5 | 296.6 |
| 527 | 24.7 | 16.3 | 45.9 |
| 473 | 503.9 | 182.0 | >3000 |

**Example 12: *In Vivo* Efficacy Study of Oral Administration in Mice**

1. Objective: The study aims to determine the *in vivo* efficacy of compound 527 by observing the inhibition effect of compound 527 on the phosphorylation level of LRRK2 in the brains of G2019S mutant LRRK2 gene knock-in mice following oral administration.
2. Method:
(1) Animals: B6.Cg-Tg(Lrrk2*G2019S)2Yue/J transgenic mice were purchased from Jackson Laboratory. The mice were bred with C57BL/6J mice to produce LRRK2 heterozygote mice carrying the G2019S mutation (LRRK2-G2019S mice). The mice included an equal number of male and female individuals, with body weights ranging from 16-20 g and ages between 15-17 weeks.
(2) Grouping and administration: The mice were randomly divided into 5 experimental groups based on their body weights. Each group consisted of 8 animals, with 4 males and 4 females. The specific administration for each experimental group is detailed in the following Table 1. Group G1 served as the vehicle control group. For group G2 to group G5, compound 527 was administered at doses of 1 mg/kg, 3 mg/kg, 10 mg/kg, and 30 mg/kg, respectively.

Table 1: Experimental grouping and route of administration

| Group | Test drug | Number of animal | Dose (mg/kg) | Route of administration | Tissue collection time (after administration) | Frequency of administration |
|---|---|---|---|---|---|---|
| G1 | Isovolumetric vehicle | 8 | - | Oral gavage | 1 h | 1 |
| G2 | 527 1mg/kg | 8 | 1 | Oral gavage | 1 h | 1 |
| G3 | 527 3mg/kg | 8 | 3 | Oral gavage | 1 h | 1 |
| G4 | 527 10mg/kg | 8 | 10 | Oral gavage | 1 h | 1 |
| G5 | 527 30mg/kg | 8 | 30 | Oral gavage | 1 h | 1 |

(3) Intracerebral LRRK2 activity assay: One hour after administration, the animals were euthanized using $CO_2$. The brain tissues were collected, rapidly frozen in liquid nitrogen, and then cryopreserved at -80 C until subsequent experiments. The brain tissues were homogenized in an RIPA buffer containing cOmplete™ protease inhibitor mixture (Roche, 04693124001) and phosphatase inhibitor mixture 2 (sigma, R0278). Protein quantification was performed using the BCA method. The phosphorylation level of LRRK2 at pS935 site (primary antibody Anti-LRRK2 (phospho S935) antibody, abcam, ab133450), and the total LRRK2 level (primary antibody Anti-LRRK2 antibody, Abcam, ab133518) were detected by Western blot method. The activity level of LRRK2 in the brain was represented by the phosphorylation level at S935 site of LRRK2, i.e., the ratio of pS935-LRRK2 to total LRRK2 (pS935-LRRK2/LRRK2).
(4) Results and conclusions: The typical results of Western Blot analysis of the brain tissues are shown in FIG. 1, and the inhibition effect of compound 527 on the phosphorylation of LRRK2 in the brain tissues is shown in FIG. 2.

The results indicated that the effective dose of compound 527 for inhibiting the phosphorylation of LRRK2 in the

brain tissues was 10 mg/kg, and the maximum effective dose (defined as 60% inhibition of the phosphorylation of LRRK2) was 30 mg/kg. The corresponding human equivalent doses (calculated for a body weight of 70 kg) were 57 mg and 171 mg, respectively.

The exemplary embodiments of the present disclosure have been described above. However, the protection scope of the present disclosure is not limited to the embodiments described above. Any modifications, equivalents, improvements and the like made by those skilled in the art without departing from the spirit and principle of the present disclosure shall fall within the protection scope of the present disclosure.

## Claims

1. A compound represented by formula (I), or a racemate, a stereoisomer, a tautomer, an isotopically labeled compound, an N-oxide, a hydrate, a solvate, a polymorph, a metabolite, a pharmaceutically acceptable salt, a pharmaceutically acceptable ester, or a prodrug compound thereof:

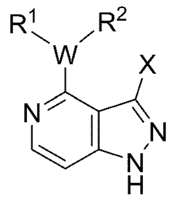
(I)

wherein,

W is selected from N or O;

$R^1$ is selected from the following groups unsubstituted or substituted with one, two, or more $R^a$: $C_{1-10}$ alkyl, $C_{3-10}$ cycloalkyl, and 3- to 10-membered heterocyclyl, wherein each $R^a$ is identical or different and is independently selected from halogen, hydroxyl, $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

or alternatively, two $R^a$, together with the atom(s) to which they are attached, form the following groups unsubstituted or optionally substituted with one, two, or more $R^c$: $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

$R^2$ is absent or selected from H or $C_{1-10}$ alkyl, wherein, when W is O, $R^2$ is absent; when W is N, $R^2$ is selected from H or $C_{1-10}$ alkyl;

or alternatively, $R^1$ and $R^2$, together with W, form 3- to 10-membered heterocyclyl unsubstituted or optionally substituted with one, two, or more $R^b$, wherein each $R^b$ is identical or different and is independently selected from halogen, hydroxyl, $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

or alternatively, two $R^b$, together with the atom(s) to which they are attached, form the following groups unsubstituted or optionally substituted with one, two, or more $R^c$: $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy, wherein each $R^c$ is identical or different and is independently selected from halogen, hydroxyl, $C_{1-10}$ alkyl, $C_{1-10}$ alkyloxy, $C_{3-10}$ cycloalkyl, $C_{3-10}$ cycloalkyloxy, 3- to 10-membered heterocyclyl, and 3- to 10-membered heterocyclyloxy;

X is selected from 5- to 10-membered heteroaryl substituted with one, two, or more $R^3$, wherein each $R^3$ is identical or different and is independently selected from the following groups unsubstituted or substituted with one, two, or more $R^4$: $C_{1-10}$ alkyl, $C_{3-10}$ cycloalkyl, 3- to 10-membered heterocyclyl, and 5- to 10-membered heteroaryl;

or alternatively, two $R^3$, together with the atom(s) to which they are attached, form the following groups unsubstituted or optionally substituted with one, two, or more $R^4$: $C_{3-10}$ cycloalkyl and 3- to 10-membered heterocyclyl;

wherein each $R^4$ is identical or different and is independently selected from halogen, hydroxyl, cyano, or the following groups unsubstituted or substituted with one, two, or more $R^d$: $C_{1-10}$ alkyl, $C_{1-10}$ alkylene, $C_{3-10}$ cycloalkyl, 3- to 10-membered heterocyclyl, and 5- to 10-membered heteroaryl, wherein when $R^4$ is selected from $C_{1-6}$ alkylene, it means that the carbon atom in $R^4$, together with the carbon atom to which $R^3$ is attached, forms a double bond;

each $R^d$ is identical or different and is independently selected from halogen, hydroxyl, cyano, $C_{1-6}$ alkyl, $C_{1-6}$ alkyloxy, $C_{3-10}$ cycloalkyl, $C_{3-10}$ cycloalkyloxy, 3- to 10-membered heterocyclyl, 3- to 10-membered heterocyclyloxy, 5- to 10-membered heteroaryl, and 5- to 10-membered heteroaryl oxy.

**2.** The compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to claim 1, wherein

each $R^a$ is identical or different and is independently selected from F, Cl, Br, or $C_{1-6}$ alkyl;
$R^1$ is selected from the following groups unsubstituted or substituted with one, two, or more $R^a$: $C_{1-8}$ alkyl, $C_{3-8}$ cycloalkyl, and 3- to 8-membered heterocyclyl; for example, $R^1$ is selected from the following groups unsubstituted or substituted with one, two, or more $R^a$: $C_{1-6}$ alkyl, $C_{3-7}$ cycloalkyl, and 3- to 7-membered heterocyclyl; for example, $R^1$ is selected from

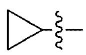, 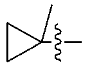, 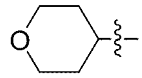,

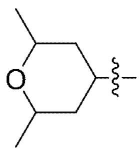,

$-CH_2CF_3$, and $-CH(CH_3)CF_3$;
alternatively, $R^1$ and $R^2$, together with W, form the following groups unsubstituted or optionally substituted with one two, or more $R^b$:

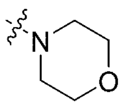, 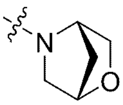, 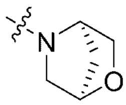, 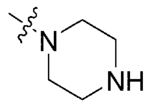, and

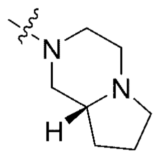;

X is selected from 5- to 6-membered heteroaryl substituted with one, two, or more $R^3$; for example, X is selected from

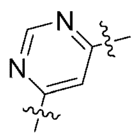 and 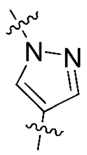

substituted with one, two, or more $R^3$; for example, X is selected from

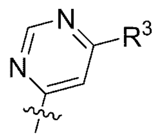 and 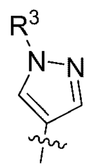;

each $R^4$ is identical or different and is independently selected from fluorine, chlorine, bromine, hydroxyl, cyano, or the following groups unsubstituted or substituted with one, two, or more $R^d$:
$C_{1-6}$ alkyl, $C_{1-6}$ alkylene, $C_{3-8}$ cycloalkyl, and 3- to 8-membered heterocyclyl;

$R^3$ is selected from the following groups unsubstituted or substituted with one, two, or more $R^4$: $C_{1-6}$ alkyl, $C_{3-8}$ cycloalkyl, 3- to 8-membered heterocyclyl, and 5- to 6-membered heteroaryl;
for example, $R^3$ is selected from the following groups unsubstituted or substituted with one two, or more $R^4$: methyl, ethyl, propyl, isopropyl, cyclopropyl, cyclobutyl,

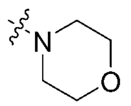, 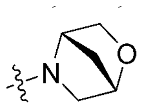,

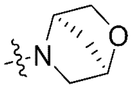, 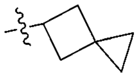, 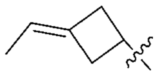, 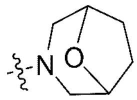, 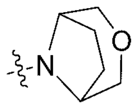, and 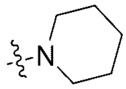;

for example, $R^3$ is selected from isopropyl,

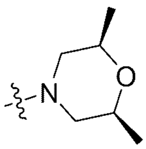, 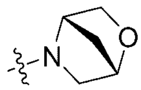, 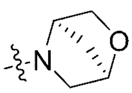, 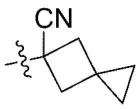, 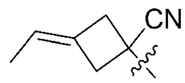,

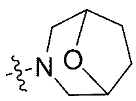, 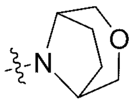, 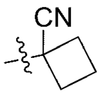, 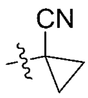, 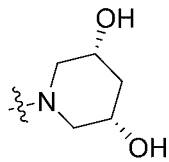, and 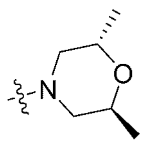.

3. The compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to claim 1 or 2, wherein the compound represented by formula (I) has a structure represented by the following formula (II):

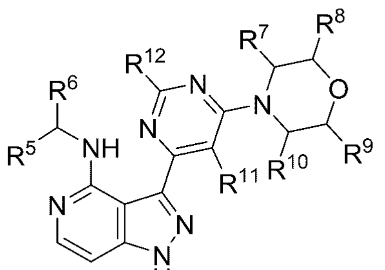

(II)

wherein $R^5$ is selected from $C_{1-6}$ alkyl substituted with one, two, or more halogens, preferably $C_{1-6}$ alkyl substituted with 1, 2, 3, 4, 5, 6, 7, 8, 9, or 10 halogens; for example, methyl or ethyl substituted with 1, 2, 3, 4, or 5 fluorine or chlorine atoms, e.g., trifluoromethyl;
$R^6$ is selected from $C_{1-6}$ alkyl, e.g., methyl, ethyl, propyl, or isopropyl;
$R^7$, $R^8$, $R^9$, and $R^{10}$ are identical or different and are each independently selected from H or $C_{1-6}$ alkyl, e.g., H, methyl, ethyl, propyl, or isopropyl; and
$R^{11}$ and $R^{12}$ are identical or different and are each independently selected from H or $C_{1-6}$ alkyl, e.g., H, methyl, ethyl, propyl, or isopropyl.

4. The compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to claim 3, wherein the compound represented by

formula (I) has a compound represented by the following formula (III):

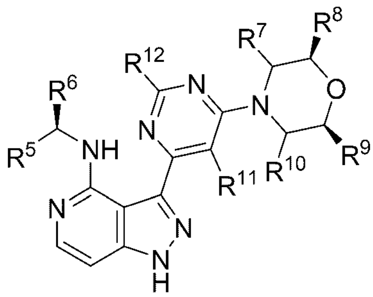

(III)

wherein $R^5$, $R^6$, $R^7$, $R^8$, $R^9$, $R^{10}$, $R^{11}$, and $R^{12}$ are as defined in claim 3.

**5.** The compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to claim 1, wherein the compound represented by formula (I) is selected from the following compounds:

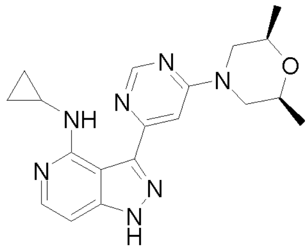

390

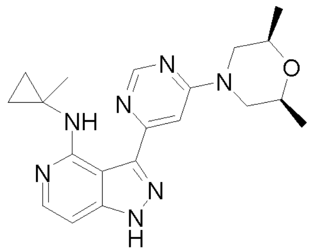

391

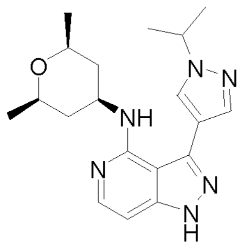

395

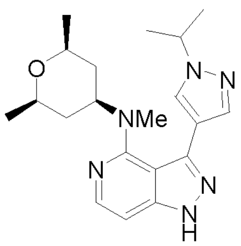

396

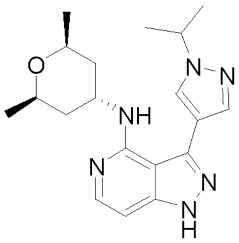

404

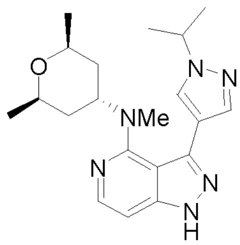

405

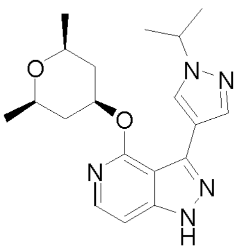

397

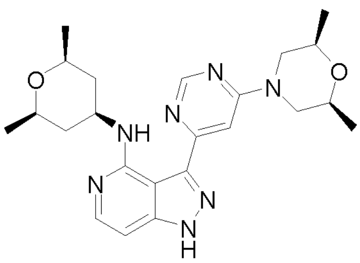

407

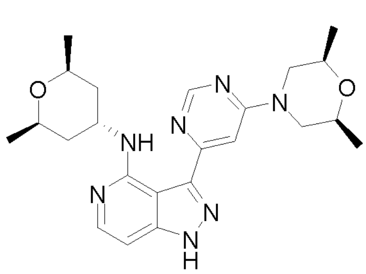

421

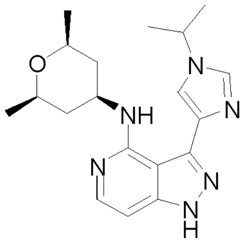

422

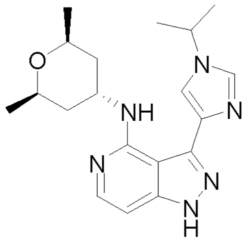

445

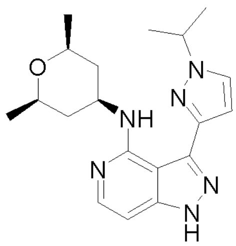

423

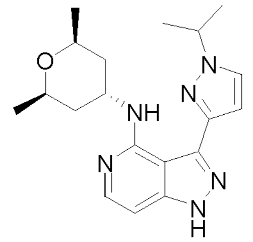

439

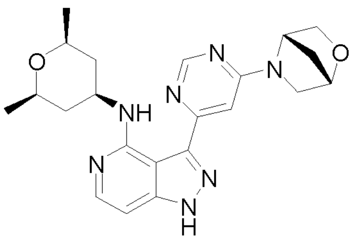

424

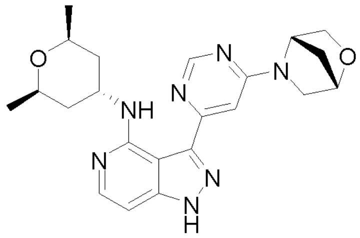

438

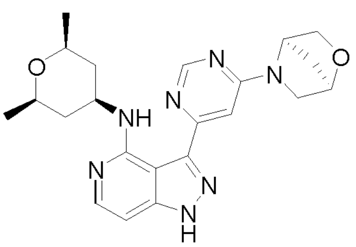

425

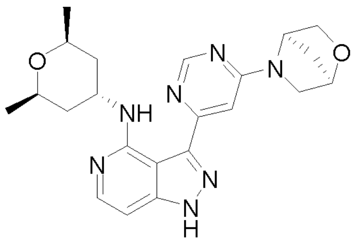

440

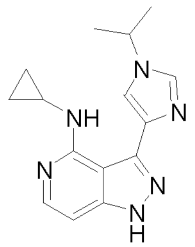

426

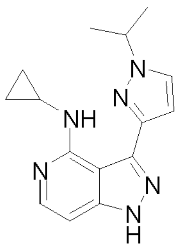

426

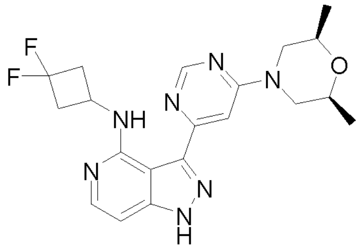

446

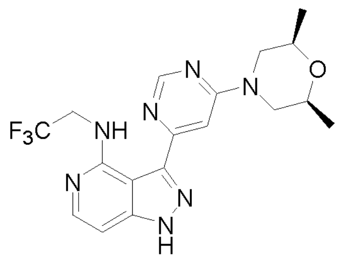

447

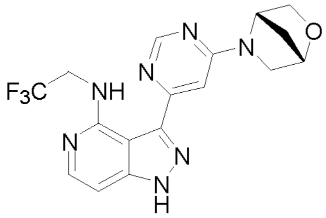

486

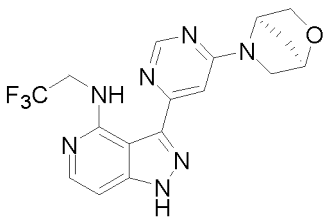

487

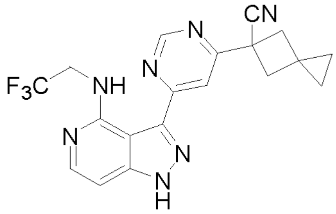

508

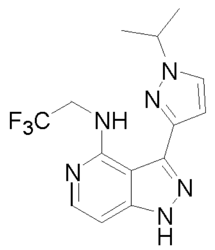
515

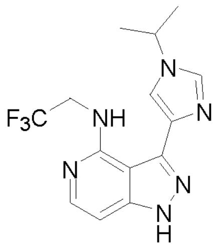
516

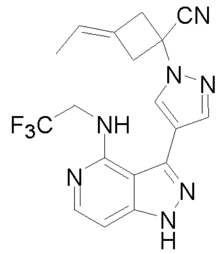
521

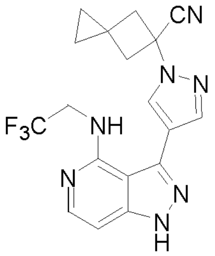
509

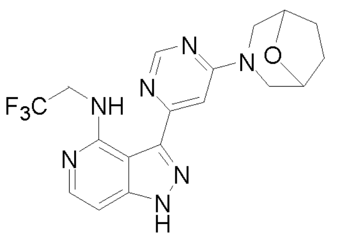
519

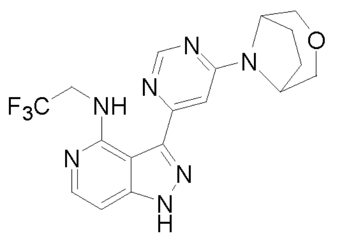
520

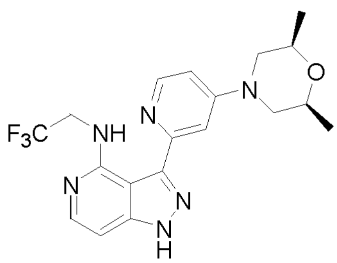
522

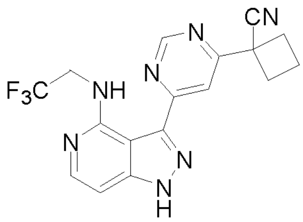
523

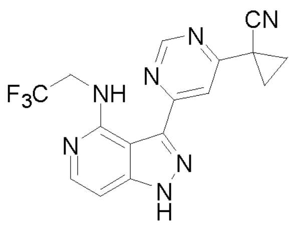
524

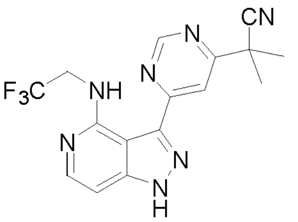
525

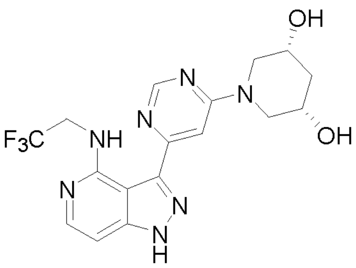
526

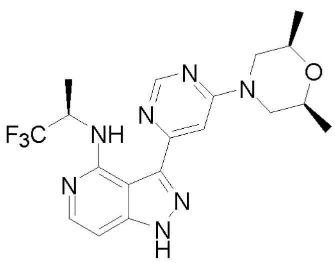
527

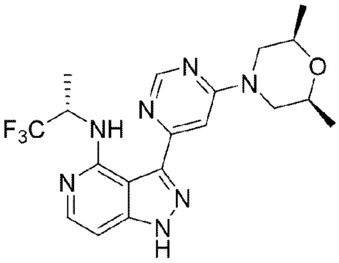
528

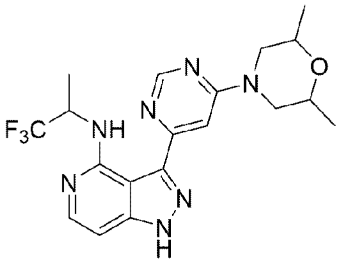
5278

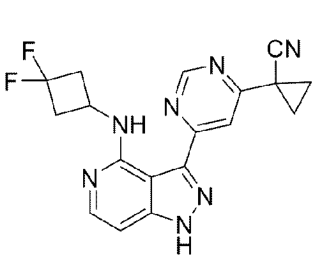
529

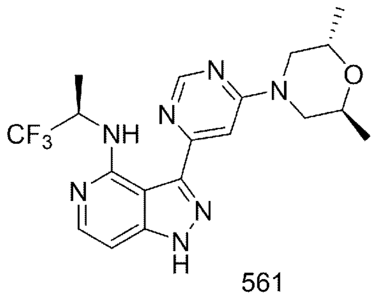
561

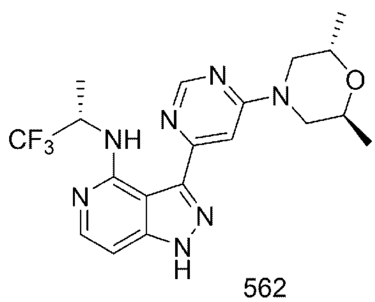
562

6. A preparation method for the compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to any one of claims 1-5, wherein the preparation method comprises the following reaction:

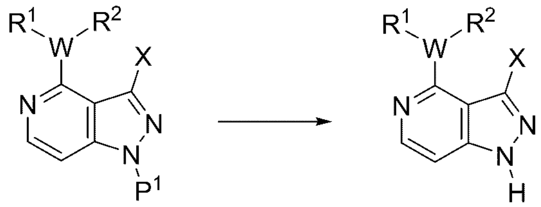
(M2) (I)

wherein $R^1$, $R^2$, W, and X are defined as above; $P^1$ is an amino protective group;
preferably, the compound represented by formula (M2) is reacted after the protective group $P^1$ is removed off to obtain the compound represented by formula (I);
preferably, $P^1$ is selected from alkyloxycarbonyl (e.g., tert-butoxycarbonyl, etc.), cycloalkylalkyloxycarbonyl (e.g., cyclohexylmethoxycarbonyl), substituted alkyloxycarbonyl (e.g., trichloroethoxycarbonyl, etc.), substituted arylalkyloxycarbonyl (e.g., *p*-nitrobenzyloxycarbonyl), arylalkyl (e.g., trityl or benzhydryl), heteroarylalkyl (e.g., pyridinylalkyl, such as 2-pyridinylmethyl and 4-pyridinylmethyl), and alkoxyarylalkyl (e.g., alkoxyphenylalkyl, such as*p*-methoxybenzyl); preferably, the preparation method further comprises the following reaction:

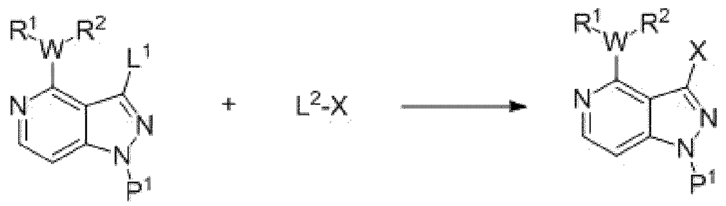
M1 M2

wherein $R^1$, $R^2$, W, X, and $P^1$ are defined as above;
$L^1$ and $L^2$ are identical or different and are each independently a leaving group capable of undergoing a coupling reaction;
preferably, the compound represented by formula (M2) is prepared through a coupling reaction known in the art, for example, through a Stille coupling reaction or a Suzuki coupling reaction;
for example, $L^1$ is selected from halogen, e.g., iodine; $L^2$ is selected from alkyl tin groups or borate groups.

7. A pharmaceutical composition comprising at least one of the compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to any one of claims 1-5, wherein

preferably, the pharmaceutical composition further comprises one or more pharmaceutically acceptable auxiliary materials;
preferably, the pharmaceutical composition is in unit dosage forms, and each unit dosage form can contain about

1-1000 mg, for example, about 5-1000 mg, more typically about 5-500 mg, e.g., 5-100 mg, 100-500 mg, or 50-200 mg of active ingredient;
preferably, the compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof is administered at doses ranging from about 1 μg/kg to about 1 g/kg body weight/day, e.g., from 0.01 mg/kg to about 100 mg/kg body weight/day;
when the subject is a human, the dose administered to the subject can be 57 mg/70 kg to 171 mg/70 kg.

**8.** Use of at least one of the compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to any one of claims 1-5 for manufacturing a medicament, wherein the medicament is used for diseases or disorders associated with the activity of LRRK kinase, particularly LRRK2 kinase.

**9.** Use of at least one of the compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to any one of claims 1-5 in analytical assays for identifying a compound capable of inhibiting one or more kinases, wherein the kinase is preferably LRRK, more preferably LRRK2;
preferably, the analytical assay is a competitive binding activity assay.

**10.** A method for detecting the binding of a compound to a kinase, wherein the method comprises the following steps:

(i) contacting at least one of the compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, or the prodrug compound thereof according to any one of claims 1-5 with a kinase in the presence of the kinase and a known substrate; and
(ii) detecting any change in the interaction between the kinase and the known substrate.

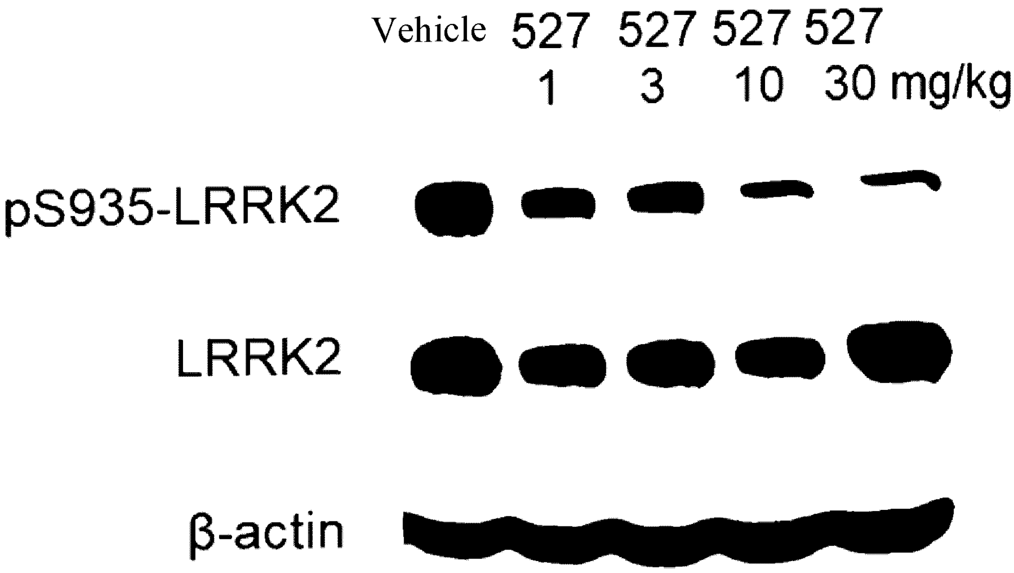
Vehicle
527 1
527 3
527 10
527 30 mg/kg
pS935-LRRK2
LRRK2
β-actin

FIG. 1

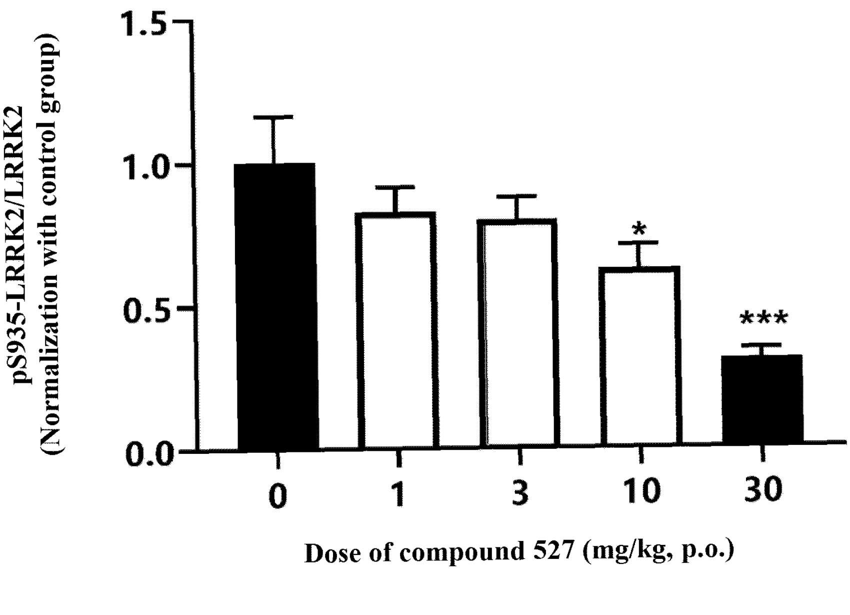
pS935-LRRK2/LRRK2
(Normalization with control group)
1.5
1.0
0.5
0.0
*
***
0
1
3
10
30
Dose of compound 527 (mg/kg, p.o.)

FIG. 2

**INTERNATIONAL SEARCH REPORT**

International application No. **PCT/CN2023/078130**

**A. CLASSIFICATION OF SUBJECT MATTER**

C07D471/04(2006.01)i;A61K31/437(2006.01)i;A61P25/16(2006.01)i;A61P25/28(2006.01)i;A61P35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI, SIPOABS, CNTXT, CNKI, REGISTRY (STN), CAPLUS (STN): 苏州亚宝, 吡唑, 吡啶, 帕金森, 癌症, PD, LRRK, 激酶, 抑制, pyrazolopyridine, pyrazolo, pyridine, Parkinson, cancer, inhibitor, kinase

**C. DOCUMENTS CONSIDERED TO BE RELEVANT**

| Category* | Citation of document, with indication, where appropriate, of the relevant passages | Relevant to claim No. |
|---|---|---|
| X | CN 102428084 A (MEDICAL RESEARCH COUNCIL TECHNOLOGY) 25 April 2012 (2012-04-25)<br>description, pages 2-4, 11-34 and 42-48 | 1-5, 7-10 |
| X | CN 103261196 A (MEDICAL RESEARCH COUNCIL TECHNOLOGY et al.) 21 August 2013 (2013-08-21)<br>description, pages 3-5, 13-15, 20-26 and 33-71 | 1-5, 7-10 |
| X | US 2014288043 A1 (GENENTECH, INC.) 25 September 2014 (2014-09-25)<br>description, pages 1-3, 5-20, 25-26 and 28-57 | 1-10 |
| X | WO 2013139882 A1 (F. HOFFMANN-LA ROCHE AG. et al.) 26 September 2013 (2013-09-26)<br>description, pages 3-6, 15-30, 46-47, 49-50 and 55-114 | 1-10 |
| X | OSBORNE, Joanne et al. "Discovery of potent and selective 5-azaindazole inhibitors of leucine-rich repeat kinase 2 (LRRK2)-Part 1"<br>*Bioorganic and Medicinal Chemistry Letters,*<br>Vol. 29, 01 December 2018 (2018-12-01), 668-673<br>pages 669-672 | 1-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 May 2023** | **16 May 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/078130**

**C. DOCUMENTS CONSIDERED TO BE RELEVANT**

| Category* | Citation of document, with indication, where appropriate, of the relevant passages | Relevant to claim No. |
|---|---|---|
| X | SHORE, Daniel G. M. et al. "Discovery of potent azaindazole leucine-rich repeat kinase 2 (LRRK2) inhibitors possessing a key intramolecular hydrogen bond-Part 2" *Bioorganic and Medicinal Chemistry Letters,* Vol. 29, 12 October 2018 (2018-10-12), 674-680 pages 676-679 | 1-8 |
| X | KHAMOULI, Saida et al. "Multi-combined 3D-QSAR, docking molecular and ADMET prediction of 5-azaindazole derivatives as LRRK2 tyrosine kinase inhibitors" *Journal of Biomolecular Structure and Dynamics,* 23 September 2020 (2020-09-23), 1-14 pages 2-4 | 1-5, 7-8 |

Form PCT/ISA/210 (second sheet) (July 2022)

**INTERNATIONAL SEARCH REPORT**
**Information on patent family members**

International application No.
**PCT/CN2023/078130**

| Patent document cited in search report | Publication date (day/month/year) | Patent family member(s) | Publication date (day/month/year) |
|---|---|---|---|
| CN 102428084 A | 25 April 2012 | MX 2011009807 A | 29 September 2011 |
| | | CA 2754605 A1 | 23 September 2010 |
| | | CA 2754605 C | 17 April 2018 |
| | | KR 20110128925 A | 30 November 2011 |
| | | KR 101700229 B1 | 26 January 2017 |
| | | AU 2010224693 A1 | 15 September 2011 |
| | | AU 2010224693 B2 | 28 July 2016 |
| | | JP 2012520861 A | 10 September 2012 |
| | | JP 5732036 B2 | 10 June 2015 |
| | | US 2017320870 A1 | 09 November 2017 |
| | | CL 2011002267 A1 | 11 May 2012 |
| | | WO 2010106333 A1 | 23 September 2010 |
| | | EP 2408772 A1 | 25 January 2012 |
| | | EP 2408772 B1 | 01 July 2015 |
| | | RU 2011142182 A | 27 April 2013 |
| | | PE 20120506 A1 | 14 May 2012 |
| | | IL 215147 A0 | 29 December 2011 |
| | | US 2010317646 A1 | 16 December 2010 |
| CN 103261196 A | 21 August 2013 | AU 2011306664 A1 | 04 April 2013 |
| | | EP 2619201 A1 | 31 July 2013 |
| | | GB 201015949 D0 | 03 November 2010 |
| | | CA 2809557 A1 | 29 March 2012 |
| | | US 2012295883 A1 | 22 November 2012 |
| | | US 8569281 B2 | 29 October 2013 |
| | | WO 2012038743 A1 | 29 March 2012 |
| | | JP 2013537218 A | 30 September 2013 |
| US 2014288043 A1 | 25 September 2014 | None | |
| WO 2013139882 A1 | 26 September 2013 | GB 201204985 D0 | 02 May 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210178818
- CN 202210470959
- WO 8403564 A


### Non-patent literature cited in the description


- **SIMON-SANCHEZ J** ; **SCHULTE C** ; **BRAS JM** ; **SHARMA M** ; **GIBBS JR et al.** Genome-wide association study reveals genetic risk underlying Parkinson's disease. *Nat Genet*, 2009, vol. 41, 1308-1312
- **WEST AB** ; **MOORE DJ** ; **BISKUP S** ; **BUGAYENKO A** ; **SMITH WW et al.** From The Cover: Parkinson's disease-associated mutations in leucine-rich repeat kinase 2 augment kinase activity. *Proceedings of the National Academy of Sciences*, 2005, vol. 102, 16842-16847
- **TAN, E. K.** Identification of a common genetic risk variant (LRRK2 Gly2385Arg) in Parkinson's disease. *Ann Acad Med Singapore*, 2006, vol. 35, 840-842
- **RUDENKO IN** ; **KAGANOVICH A** ; **HAUSER DN** ; **BEYLINA A** ; **CHIA R et al.** The G2385R variant of leucine-rich repeat kinase 2 associated with Parkinson's disease is a partial loss-of-function mutation. *Biochemical Journal*, 2012, vol. 446, 99-111
- **REINHARDT P** ; **SCHMID B** ; **BURBULLA LENA F** ; **SCHÖNDORF DAVID C** ; **WAGNER L et al.** Genetic Correction of a LRRK2 Mutation in Human iPSCs Links Parkinsonian Neurodegeneration to ERK-Dependent Changes in Gene Expression. *Cell Stem Cell*, 2013, vol. 12, 354-367
- Handbook of Pharmaceutical Excipients. 1994
- **A.R. GENNARO**. Remington's Pharmaceutical Sciences. Mack Publishing Co., 1985
- **BERGE**. *J Pharm Sci.*, 1977, vol. 66, 199